# EUROPEAN PATENT APPLICATION

(11) **EP 4 349 983 A1**
(43) Date of publication of application: **10.04.2024**
(21) Application number: 22811049.0
(22) Date of filing: 08.04.2022
(51) Int. Cl.: C12N 15/09, A01H 1/00, A01K 67/027, C12N 5/10, C12N 15/11

(54) **GENOME EDITING METHOD**

(30) Priority: 27.05.2021 JP 2021089110
(71) Applicant: National Institute of Advanced Industrial Science and Technology, Chiyoda-ku Tokyo 100-8921 (JP)
(72) Inventor: MASEDA, Hideaki, Ikeda-shi, Osaka 563-8577 (JP)
(74) Representative: Wasner, Marita
(86) International application number: PCT/JP2022/017353
(87) International publication number: WO 2022/249775

(57) **Abstract**

The present invention provides: a single-strand form polynucleotide for promoting editing and to be combined with a single-strand form polynucleotide for editing and capable of modifying a target site in double-strand genomic DNA; a genome editing kit that includes said polynucleotide for editing or an expression vector therefor, and said polynucleotide for promoting editing or an expression vector therefor; a method for modifying a target site in double-strand genomic DNA of a cell or a non-human organism, the method comprising a step for treating the cell or the organism using the genome editing kit; and a method for producing a cell or an organism in which a target site in double-strand genomic DNA is modified, the method comprising said step.

## Description

### Field

The present invention relates to a method for genome editing.

### Background

In recent years, genome editing tools such as zinc finger nucleases (ZFNs), TALENs, and CRISPR/Cas systems have been developed, and there has been active global engagement in genome editing using these tools. While ZFNs and TALENs have the disadvantage of requiring laborious design for sequence-specific nucleases, CRISPR/Cas systems have been adopted as genome editing tools especially worldwide because they are extremely easy for targeting. However, the difficulties of CRISPR/Cas systems include the problem of off-targeting and the less easy delivery of giant nucleases to cells. These genome editing technologies edit genomes using endonucleases, which are enzymes that cleave DNA, and thus require the size and design of nucleases.

In contrast, a method for genome editing using single-stranded polynucleotides alone has been developed (Patent Literatures 1 and 2). In the method of Patent Literatures 1 and 2, genome editing can be performed using single-stranded polynucleotides alone, without introducing components other than the single-stranded polynucleotides into the cell. In the method of Patent Literatures 1 and 2, genome editing can be performed not only in prokaryotic cells but also in eukaryotic cells.

### Citation List

### Patent Literature

Patent Literature 1: Japanese Patent No. 6899564
Patent Literature 2: Japanese Patent Application Publication No. 2016-220639

### Summary

The present invention provides a method for editing a genome. The present invention provides a method for editing a genome in a cell, particularly in a eukaryotic cell.

In the course of studying various formulations for enhancing the genome-editing efficiency of single-stranded polynucleotides, the inventor of the present invention surprisingly has found that genome-editing efficiency is improved in the presence of certain single-stranded antisense polynucleotides, leading to the present invention. That is, the inventor synthesized single-stranded antisense polynucleotides (also referred to as "promoting antisense polynucleotides for genome editing" or "promoting antisense polynucleotides") and tested the genome-editing efficiency by single-stranded polynucleotides for genome editing (also referred to as "genome-editing sense polynucleotides"), in the presence of the promoting antisense polynucleotides. When the promoting antisense polynucleotides were designed to have a sequence that is complementary to or has complementarity with the terminal part of the genome-editing sense polynucleotides (i.e., they are designed to bind to the terminal of the genome-editing sense polynucleotides), we found that the genome-editing efficiency is improved. Although not bound by theory, when a genomic region that is transcribed into RNA is the target to be edited, competition can occur between the transcribed RNAs around the genomic DNA and the genome-editing sense polynucleotides with respect to binding to the genomic DNA. The above-mentioned improvement in genome-editing efficiency is thought to be due to the suppression of the competition as a result of the binding of the promoting antisense polynucleotides to the RNAs. In genome editing technologies, the technical concept of enhancing genome-editing efficiency by suppressing such competition for binding to genomic DNA has never existed before, and the present invention is based on this technical concept.

The present disclosure provides the followings.
Item 1A. A single-stranded promoting polynucleotide for genome editing for use in combination with a single-stranded genome-editing polynucleotide capable of modifying a target site on a double-stranded genomic DNA,
   wherein the target site consists of a primary editing site set on one genomic DNA strand and a secondary editing site on the other genomic DNA strand at a position corresponding to the primary editing site,
   the genome-editing polynucleotide consists of, in order from its 5' terminal side,
      a) a portion capable of hybridizing to the region adjacent to the 3' terminal side of the primary editing site,
      x) an optional portion consisting of a nucleotide sequence that is not identical to the nucleotide sequence of the secondary editing site, and
      b) a portion capable of hybridizing to the region adjacent to the 5' terminal side of the primary editing site, or
   the genome-editing polynucleotide consists of, in order from its 5' terminal side, portion a) and portion b),
   the promoting polynucleotide for genome editing is capable of hybridizing to a nucleic acid sequence that is complementary to a predetermined region on the one genomic DNA strand,
   the predetermined region overlaps at its 5' terminal part with region A or overlaps at its 3' terminal part with region B on the one genomic DNA strand, in which a region on the one genomic DNA strand at a position corresponding to portion a) of the genome-editing polynucleotide is defined as region A, and a region on the one genomic DNA strand at a position corresponding to portion b) of the genome-editing polynucleotide is defined as region B,
   the length of overlap with region A at the 5' terminal part of the predetermined region is the same as or shorter than region A, and the length of overlap with region B at the 3' terminal part of the predetermined region is the same as or shorter than region B, and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
Item 1B. A single-stranded promoting polynucleotide for genome editing for use in combination with a single-stranded genome-editing polynucleotide capable of modifying a target site on a double-stranded genomic DNA,
   wherein the target site consists of a primary editing site set on one genomic DNA strand and a secondary editing site on the other genomic DNA strand at a position corresponding to the primary editing site,
   the genome-editing polynucleotide consists of, in order from its 5' terminal side,
      a) a portion consisting of a nucleotide sequence having 90% or more identity with the nucleotide sequence of the region adjacent to the 5' terminal side of the secondary editing site,
      x) an optional portion consisting of a nucleotide sequence that is not identical to the nucleotide sequence of the secondary editing site, and
      b) a portion consisting of a nucleotide sequence having 90% or more identity with the nucleotide sequence of the region adjacent to the 3' terminal side of the secondary editing site, or
   the genome-editing polynucleotide consists of, in order from its 5' terminal side, portion a) and portion b),
   the promoting polynucleotide for genome editing consists of a nucleotide sequence having 90% or more identity with the nucleotide sequence of a predetermined region on the one genomic DNA strand,
   the predetermined region overlaps at its 5' terminal part with region A or overlaps at its 3' terminal part with region B on the one genomic DNA strand, in which a region on the one genomic DNA strand at a position corresponding to portion a) of the genome-editing polynucleotide is defined as region A, and a region on the one genomic DNA strand at a position corresponding to portion b) of the genome-editing polynucleotide is defined as region B,
   the length of overlap with region A at the 5' terminal part of the predetermined region is the same as or shorter than region A, and the length of overlap with region B at the 3' terminal part of the predetermined region is the same as or shorter than region B, and
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
Item 1C. A single-stranded promoting polynucleotide for genome editing for use in combination with a single-stranded genome-editing polynucleotide capable of modifying a target site on a double-stranded genomic DNA,
   wherein the target site consists of a primary editing site set on one genomic DNA strand and a secondary editing site on the other genomic DNA strand at a position corresponding to the primary editing site,
   the genome-editing polynucleotide consists of, in order from its 5' terminal side,
      a) a portion consisting of a nucleotide sequence identical to the nucleotide sequence of the region adjacent to the 5' terminal side of the secondary editing site,
      x) an optional portion consisting of a nucleotide sequence that is not identical to the nucleotide sequence of the secondary editing site, and
      b) a portion consisting of a nucleotide sequence identical to the nucleotide sequence of the region adjacent to the 3' terminal side of the secondary editing site, or
   the genome-editing polynucleotide consists of, in order from its 5' terminal side, portion a) and portion b),
   the promoting polynucleotide for genome editing consists of a nucleotide sequence identical to the nucleotide sequence of a predetermined region on the one genomic DNA strand,
   the predetermined region overlaps at its 5' terminal part with region A or overlaps at its 3' terminal part with region B on the one genomic DNA strand, in which a region on the one genomic DNA strand at a position corresponding to portion a) of the genome-editing polynucleotide is defined as region A, and a region on the one genomic DNA strand at a position corresponding to portion b) of the genome-editing polynucleotide is defined as region B,
   the length of overlap with region A at the 5' terminal part of the predetermined region is the same as or shorter than region A, and the length of overlap with region B at the 3' terminal part of the predetermined region is the same as or shorter than region B,
   when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site, and
   when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
   when the genome-editing polynucleotide consists of portion a) and portion b), and the primary editing site is set at one nucleotide or multiple contiguous nucleotides on the one genomic DNA strand, the genome-editing polynucleotide can delete the target site.
Item. 2A. The promoting polynucleotide for genome editing according to item 1A, 1B or 1C, wherein the length of the promoting polynucleotide for genome editing is 50 nucleotides or more.
Item. 2B. The promoting polynucleotide for genome editing according to item 1A, 1B, 1C or 2A, wherein the length of the overlap is 5 nucleotides or more.
Item. 3A. A kit for genome editing, containing:
   the promoting polynucleotide for genome editing according to item 1A, 2A or 2B,
   an expression vector thereof, or
   a conjugate of the promoting polynucleotide for genome editing or the expression vector thereof with one or more other substances, and
   a genome-editing polynucleotide,
   an expression vector thereof, or
   a conjugate of the genome-editing polynucleotide or the expression vector thereof with one or more other substances,
   wherein the genome-editing polynucleotide consists of, in order from its 5' terminal side,
      a) a portion capable of hybridizing to the region adjacent to the 3' terminal side of the primary editing site,
      x) an optional portion consisting of a nucleotide sequence that is not identical to the nucleotide sequence of the secondary editing site, and
      b) a portion capable of hybridizing to the region adjacent to the 5' terminal side of the primary editing site, or
   the genome-editing polynucleotide consists of, in order from its 5' terminal side, portion a) and portion b).
Item. 3B. A kit for genome editing, containing:
   the promoting polynucleotide for genome editing according to item 1B, 2A or 2B,
   an expression vector thereof, or
   a conjugate of the promoting polynucleotide for genome editing or the expression vector thereof with one or more other substances, and
   a genome-editing polynucleotide,
   an expression vector thereof, or
   a conjugate of the genome-editing polynucleotide or the expression vector thereof with one or more other substances,
   wherein the genome-editing polynucleotide consists of, in order from its 5' terminal side,
      a) a portion consisting of a nucleotide sequence having 90% or more identity with the nucleotide sequence of the region adjacent to the 5' terminal side of the secondary editing site,
      x) an optional portion consisting of a nucleotide sequence that is not identical to the nucleotide sequence of the secondary editing site, and
      b) a portion consisting of a nucleotide sequence having 90% or more identity with the nucleotide sequence of the region adjacent to the 3' terminal side of the secondary editing site, or
   the genome-editing polynucleotide consists of, in order from its 5' terminal side, portion a) and portion b).
Item. 3C. A kit for genome editing, containing:
   the promoting polynucleotide for genome editing according to item 1C, 2A or 2B,
   an expression vector thereof, or
   a conjugate of the promoting polynucleotide for genome editing or the expression vector thereof with one or more other substances, and
   a genome-editing polynucleotide,
   an expression vector thereof, or
   a conjugate of the genome-editing polynucleotide or the expression vector thereof with one or more other substances,
   wherein the genome-editing polynucleotide consists of, in order from its 5' terminal side,
      a) a portion consisting of a nucleotide sequence identical to the nucleotide sequence of the region adjacent to the 5' terminal side of the secondary editing site,
      x) an optional portion consisting of a nucleotide sequence that is not identical to the nucleotide sequence of the secondary editing site, and
      b) a portion consisting of a nucleotide sequence identical to the nucleotide sequence of the region adjacent to the 3' terminal side of the secondary editing site, or
   the genome-editing polynucleotide consists of, in order from its 5' terminal side, portion a) and portion b).
Item 4. The kit for genome editing according to item 3A, 3B or 3C, containing:
   the promoting polynucleotide for genome editing in which the predetermined region is set to overlap at its 5' terminal part with region A,
   an expression vector thereof, or
   a conjugate of the promoting polynucleotide for genome editing or the expression vector thereof with one or more other substances, and
   the promoting polynucleotide for genome editing in which the predetermined region is set to overlap at its 3' terminal part with region B,
   an expression vector thereof, or
   a conjugate of the promoting polynucleotide for genome editing or the expression vector thereof with one or more other substances.
Item 5. The kit for genome editing according to item 3A, 3B, 3C or 4, wherein the length of the overlap with respect to the promoting polynucleotide for genome editing is 5 nucleotides or more.
Item 6. A method for modifying a target site on a double-stranded genomic DNA of a cell or an organism, including the step of treating the cell or the organism using the kit for genome editing according to item 3A, 3B, 3C, 4 or 5.
Item 7. A method for producing a cell or an organism in which a target site on a double-stranded genomic DNA has been modified, including the step of treating the cell or the organism using the kit for genome editing according to item 3A, 3B, 3C, 4 or 5.

### Advantageous Effects of Invention

According to the present invention, genome editing can be performed efficiently.

### Brief Description of Drawings

[FIG.1] Fig. 1 is an image illustrating the design of polynucleotides (1) and (2) that are genome-editing polynucleotides (sense polynucleotides), and polynucleotides (3) to (16) and (22) to (27) that are promoting polynucleotides for genome editing (antisense polynucleotides) used in the Examples.
[FIG. 2] Fig. 2 is a graph illustrating changes in the efficiency of intron-targeted genome editing by genome-editing polynucleotides in the presence of some of polynucleotides (3) to (16) that are promoting polynucleotides for genome editing (anti-sense polynucleotides).
[FIG. 3] Fig. 3 is a graph illustrating changes in the efficiency of intron-targeted genome editing by genome-editing polynucleotides in the presence of some of polynucleotides (11) to (16) and (22) to (27) that are promoting polynucleotides for genome editing (anti-sense polynucleotides).
[FIG. 4] Fig. 4 is an image illustrating the design of polynucleotides (18), (17) and (1) that are genome-editing polynucleotides (sense polynucleotides), and polynucleotides (3) and (4) that are promoting polynucleotides for genome editing (antisense polynucleotides) used in the Examples.
[FIG. 5] Fig. 5 illustrates that the genome-editing efficiency is improved in the presence of a promoting polynucleotide for genome editing (antisense polynucleotide) in both cases of using a polynucleotide (18) that is a genome-editing polynucleotide (sense polynucleotide) with base deletion in the sequence, and of using a polynucleotide (17) that is a genome-editing polynucleotide (sense polynucleotide) with base insertion in the sequence.
[FIG. 6] Fig. 6 is an image illustrating the design of polynucleotide (19) that is a genome-editing polynucleotide (sense polynucleotide) targeting an exon, and polynucleotides (20) and (21) that are promoting polynucleotides for genome editing (antisense polynucleotides) used in the Examples.
[FIG. 7] Fig. 7 illustrates that the genome-editing efficiency with genome-editing sense polynucleotide is greatly improved in the presence of even one type of promoting polynucleotide for genome editing (antisense polynucleotide).
[FIG. 8A] Fig. 8A is an image illustrating the mechanism of conventional genome editing (substitution) by a genome-editing polynucleotide.
[FIG. 8B] Fig. 8B is an image illustrating the mechanism of genome editing (substitution) by a genome-editing polynucleotide and two types of promoting polynucleotides for genome editing.
[FIG. 9A] Fig. 9A is an image illustrating the mechanism of conventional genome editing by a genome-editing sense polynucleotide.
[FIG. 9B] Fig. 9B is an image illustrating the mechanism of genome editing by a genome-editing sense polynucleotide in the presence of promoting antisense polynucleotides for genome editing.

### Detailed Description of Invention

The following description may be based on representative embodiments or specific examples, but the present invention is not limited to such embodiments or specific examples. The upper limit value and the lower limit value of each numerical range exemplified in the present specification can be combined as desired. In the present specification, a numerical range represented using "to" or "-" means a range including numerical values at both ends thereof as an upper limit value and a lower limit value, unless otherwise noted.

In the present disclosure, the term "genome editing" or similar terms refer to a type of method of modifying a genome. Genome editing requires targeting a specific region on a genome and selectively modifying the region.
In recent years, genome editing tools such as zinc finger nucleases (ZFNs), TALENs, and CRISPR/Cas systems have been developed, and there has been active global engagement in genome editing using these tools. While ZFNs and TALENs have the disadvantage of requiring laborious design for sequence-specific nucleases, CRISPR/Cas systems have been adopted as genome editing tools especially worldwide because they are extremely easy for targeting. However, the difficulties of CRISPR/Cas systems include the problem of off-targeting and the less easy delivery of giant nucleases to cells.

In the present disclosure, a "cell" refers to a basic structural and functional unit of an organism. A cell is a basic unit of a living organism that has at least genomic DNA, cytoplasm, and a membrane structure enclosing them. A cell can be a eukaryotic cell or a prokaryotic cell. A eukaryotic cell has a nucleus surrounded by a nuclear membrane, and cytoplasm. A prokaryotic cell does not have a nucleus. Genomic DNA includes the endogenous DNA of the cell, but does not necessarily consist solely of the cell's endogenous factors. A eukaryotic cell can be a human cell. A cell can be a purified cell or an isolated cell. A cell can be a cultured cell. A cell can be an established cell. A cell can be, for example, a somatic cell. A cell can be, for example, a pluripotent cell or a pluripotent stem cell. A cell can be, for example, a tissue stem cell. A cell can be, for example, a tissue progenitor cell. A cell can be, for example, a germ cell.

An organism composed of eukaryotic cells is called a eukaryote, while an organism composed of prokaryotic cells is called a prokaryote. Eukaryotes include animals, plants, and fungi. Animals include mammals (human and non-human mammals), especially domestic animals, such as cattle, pigs, goats, sheep, horses, llamas, and camels; birds, such as chickens; agricultural animals, such as fish and other seafood; pet animals, such as dogs, cats, rabbits, guinea pigs, hamsters, and mice; vertebrates, such as birds, fish, amphibians, and reptiles; insects; crustaceans, such as crabs, and shrimp; shellfish; and mollusks, such as octopuses, and squids. Plants include agricultural crop plants, e.g., food crop plants, e.g., rice, corn, potatoes, beans, barley, wheat, and similar grains, horticultural crop plants (vegetables, fruit trees, and flowers), e.g., leaf vegetables (cabbage, asparagus, etc.), fruit vegetables (eggplant, tomato, cucumber, etc.), root vegetables (radish, carrot, etc.), other vegetables, fruits (e.g., pome fruits such as apple and pear, stone fruits such as Japanese apricot, apricot, plum, peach, and cherry, nuts such as almonds, walnuts, and chestnuts, and citrus fruits such as tangerines and lemons, and tropical fruit trees such as tropical fruits), and ornamental plants, as well as seeds, seedlings, or bulbs of any of these. Another example of eukaryotes is fungi such as yeast, molds, and basidiomycetes, examples of which can include yeast, red bread mold, matsutake mushroom, shiitake mushroom, enoki mushroom, shimeji mushroom, nameko mushroom, and eryngii mushroom. A eukaryote can also be a microalgae. A eukaryote can be human or non-human.

Prokaryotes include, for example, Gram-negative bacteria (Pseudomonas aeruginosa, Escherichia coli, Serratia spp., Sphingomonas spp., Brucella spp., Neisseria gonorrhoeae, Burkholderia spp., Shigella spp., Salmonella spp., Acinetobacter spp., Vibrio cholerae, Klebsiella pneumoniae, Legionella spp., Helicobacter pylori, Campylobacter spp., etc.), Gram-positive bacteria (Mycobacterium tuberculosis, Mycoplasma spp., Staphylococcus aureus, Actinomyces, Bacillus subtilis, Bacillus anthracis, etc.). Prokaryotes can preferably be Gram-negative bacteria, more preferably Pseudomonas aeruginosa, Escherichia coli, Serratia spp. or Sphingomonas spp, even more preferably Pseudomonas aeruginosa or Escherichia coli.

A cell contains the genomic DNA of the cell, and may additionally contain the genomic DNA of a foreign invader, such as a pathogen. In the present disclosure, unless otherwise noted, genomic DNA refers to the genomic DNA of the cell itself, that is, the host genomic DNA. The genomic DNA of the invader might exist in the cell as a separate entity from the host genomic DNA, or might be incorporated into the genomic DNA of the host. The host genomic DNA may contain exogenous factors (e.g., insertion of whole or part of the genomic DNA of the invader, such as a virus). Host genomic DNA that incorporates the genomic DNA of an invader, as well as host genomic DNA that contains an exogenous factor are also encompassed in genomic DNA as used herein.

In the present disclosure, "isolation" means separating cell(s) of interest from at least one other component. Isolation can be performed, for example, by separating and extracting the cell(s) in their natural state of existence from other components that exist together in the natural state of existence. Isolation can be performed, for example, by separating and extracting a portion of cells from a multicellular organism. In the present disclosure, a technique for handling isolated cells is called an in vitro technique.

In the present disclosure, "purification" means further separating the isolated cell(s) of interest from other components with which they coexist. Purification can be performed, for example, by separating the cell(s) of interest from other components based on morphology or surface markers. Purification can be performed by limiting dilution and/or cloning of the cell(s). Purification can be performed by establishing a cell line of the cell(s) of interest. Purification can be performed based on the expression of marker genes, such as drug resistance genes or genes encoding fluorescent proteins, if the cell(s) of interest have such marker genes.

In the present disclosure, the terms "polynucleotide" and "nucleic acid" can be used interchangeably and refer to nucleotide polymers that are linked by phosphodiester bonds. "Polynucleotide" and "nucleic acid" may be DNA, RNA, or may be composed of a combination of DNA and RNA (e.g., a gapmer). "Polynucleotide" and "nucleic acid" may also be polymers of natural nucleotides, or of natural nucleotides and non-natural nucleotides (analogs of natural nucleotides, nucleotides in which at least one of the base, sugar and phosphate parts is modified (e.g., phosphorothioate backbone), etc.), or may be polymers of non-natural nucleotides. A polynucleotide may be a peptide nucleic acid. A polynucleotide may be a morpholino oligo.

In the present disclosure, unless otherwise indicated, the nucleotide sequence of "polynucleotide" or "nucleic acid" is described using generally accepted single letter codes. Unless otherwise indicated, nucleotide sequences are described from the 5' side to the 3' side. Nucleotide residues constituting " polynucleotide" or " nucleic acid" may be described simply as adenine, thymine, cytosine, guanine, or uracil, etc., or by their single letter codes.

In the present disclosure, the term "gene" refers to a polynucleotide that contains at least one open reading frame encoding a certain protein. A gene can include both exon(s) and intron(s). The term "region where RNA is transcribed" includes regions where RNA is transcribed, including regions where a gene is present and regions where non-coding RNA is transcribed. The region where RNA is transcribed can be a region that exhibits RNA transcription activity. From the region where RNA is transcribed, RNA is transcribed using the minus strand as a template. The minus strand is referred to as the antisense strand, and the complementary strand of the minus strand is referred to as the sense strand.

The term "operably linked" as used with respect to a polynucleotide means that a regulatory sequence, such as a promoter, is located sufficiently close to a gene sequence that can influence the expression of the gene sequence. For example, "operably linked to a promoter" with respect to a polynucleotide means that the polynucleotide is linked such that it is expressed under the control of the promoter.

The term "expressible" means that a polynucleotide is in a state in which the polynucleotide can be transcribed in the cell into which the polynucleotide is introduced.

The term "expression vector" refers to a vector that contains a polynucleotide of interest and is equipped with a mechanism to express the polynucleotide of interest in the cell into which the vector is introduced. For example, "polynucleotide expression vector" means a vector capable of expressing a polynucleotide in a cell into which the vector is introduced. In a polynucleotide expression vector, the polynucleotide is operably linked to a regulatory sequence, for example.

In the present disclosure, nucleotide sequence identity (or homology) is determined as the percentage of matching bases across the entire sequence, excluding gaps in the alignment obtained by aligning two nucleotide sequences to maximize base matches, introducing gaps where insertions and deletions occur. Nucleotide sequence identity can be determined using various homology search software known in the art. For example, the sequence identity of nucleotide sequences can be obtained by calculation based on alignment obtained by BLASTN, a known homology search software. Here, when a sequence to be aligned contains uracil, the identity calculation is performed using the sequence where uracil has been replaced with thymine. When a sequence to be aligned contains a modified nucleotide or nucleotide analog, the identity calculation is performed using the sequence where the modified nucleotide or nucleotide analog has been replaced with its corresponding natural deoxyribonucleotide, i.e. adenine, thymine, guanine or cytosine.

The terms " portion" and "region" with respect to a polynucleotide can be used interchangeably and both mean a one nucleotide or multiple contiguous nucleotides contained in the polynucleotide. The terms "portion" and "region" with respect to double-stranded genomic DNA can be used interchangeably and both mean a one nucleotide pair or multiple contiguous nucleotide pairs contained in the double-stranded genomic DNA.

In the present disclosure, a portion or region "at a position corresponding to" a portion or region of a polynucleotide refers to a portion or region complementary to the portion or region of that polynucleotide on a different polynucleotide.

In the present disclosure, a polynucleotide or a portion or region thereof that is " capable of hybridizing" to another polynucleotide or a portion or region thereof means that one polynucleotide or a portion or region thereof has the ability to bind to the other polynucleotide or a portion or region thereof through hydrogen bonds between complementary bases. The capability to hybridize can be confirmed by maintaining the hybridized state under a stringent condition. As taught in Berger and Kimmel (1987, Guide to Molecular Cloning Techniques Methods in Enzymology, Vol. 152, Academic Press, San Diego CA), a stringent condition can be determined based on the melting temperature (Tm) of a nucleic acid to which a probe is to be bound. When the hybridization state between two polynucleotides or their portions or regions is maintained by washing under a stringent condition, e.g., with 1 x SSC, 0.1 % SDS, at 37°C, they are capable of hybridizing to each other. A stringent condition may be washing with 0.5 x SSC, 0.1 % SDS, at 42°C, or with 0.1 x SSC, 0.1 % SDS, at 65°C.

### Genome-editing polynucleotide

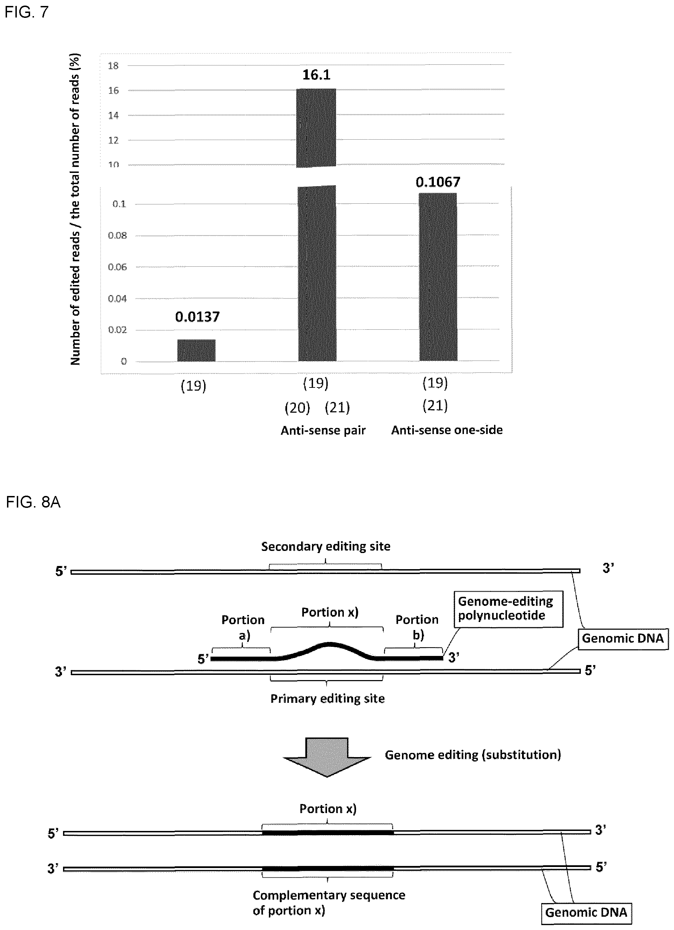

In the present disclosure, a genome-editing polynucleotide is a single-stranded polynucleotide capable of modifying a target site on a double-stranded genomic DNA. The genome-editing polynucleotide consists of, in order from its 5' terminal side,
a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b). The details of the genome-editing polynucleotide are described below (see also Fig. 8A).

In the present disclosure, a target site refers to one nucleotide pair or multiple contiguous nucleotide pairs, or a position between two contiguous nucleotide pairs, on a double-stranded genomic DNA that is intended to be modified. A target site consists of a primary editing site set on one genomic DNA strand and a secondary editing site on the other genomic DNA strand at a position corresponding to the primary editing site. For example, when the intended modification is to replace or delete one nucleotide pair or multiple contiguous nucleotide pairs in double-stranded genomic DNA, the primary editing site is set at one nucleotide or multiple nucleotides intended to be replaced or deleted on one genomic DNA strand, when the intended modification is to insert one nucleotide pair or multiple contiguous nucleotide pairs into double-stranded genomic DNA, the primary editing site is set between two contiguous nucleotides into which one nucleotide or multiple contiguous nucleotides intended to be inserted on one genomic DNA strand.

The primary editing site can be set at any one nucleotide or multiple contiguous nucleotides, or between any two contiguous nucleotides, on one genomic DNA strand. The primary editing site is not limited to being set at a specific one nucleotide or multiple contiguous nucleotides, or between two specific contiguous nucleotides.

When the primary editing site is set at one nucleotide or multiple contiguous nucleotides, the region adjacent to the 5' terminal side of the primary editing site and the region adjacent to the 3' terminal side of the primary editing site are separated by the length of the primary editing site on the genomic DNA. When the primary editing site is set at a position between two contiguous nucleotides, the region adjacent to the 5' terminal side of the primary editing site and the region adjacent to the 3' terminal side of the primary editing site are adjacent to each other on the genomic DNA.

When the primary editing site is set at multiple contiguous nucleotides on one genomic DNA strand, the number of contiguous nucleotides is not particularly limited, and can be, for example, 2 nucleotides or more, 3 nucleotides or more, 4 nucleotides or more, 5 nucleotides or more, 6 nucleotides or more, 7 nucleotides or more, or 8 nucleotides or more, and can be, for example, 2000 nucleotides or less, 1000 nucleotides or less, 900 nucleotides or less, 800 nucleotides or less, 700 nucleotides or less, 600 nucleotides or less, 500 nucleotides or less, 450 nucleotides or less, 400 nucleotides or less, 350 nucleotides or less, 300 nucleotides or less, 250 nucleotides or less, 200 nucleotides or less, 150 nucleotides or less, 100 nucleotides or less, 90 nucleotides or less, 80 nucleotides or less, 70 nucleotides or less, 60 nucleotides or less, 50 nucleotides or less, 40 nucleotides or less, 30 nucleotides or less, 20 nucleotides or less, 15 nucleotides or less, 12 nucleotides or less, 10 nucleotides or less, or 9 nucleotides or less.

The primary editing site may be set on either strand of the two strands constituting genomic DNA. The secondary editing site is to be set on the strand of genomic DNA on which the primary editing site is not set. For example, when modification of the region of double-stranded genomic DNA that is transcribed into RNA is intended, the primary editing site may be set on the sense strand or on the antisense strand.

Portion a) of the genome-editing polynucleotide can hybridize with the region adjacent to the 3' terminal side of the primary editing site.

Portion a) can also be a portion consisting of a nucleotide sequence having 90% or more identity with the nucleotide sequence of the region adjacent to the 5' terminal side of the secondary editing site. Here, the identity is preferably 95% or more, more preferably 97% or more, further more preferably 99% or more, even more preferably 99.5% or more, and especially preferably 99.9% or more.

In a preferred embodiment, portion a) consists of a nucleotide sequence complementary to the nucleotide sequence of the region adjacent to the 3' terminal side of the primary editing site, i.e., a nucleotide sequence identical to the nucleotide sequence of the region adjacent to the 5' terminal side of the secondary editing site.

Portion b) of the genome-editing polynucleotide can hybridize with the region adjacent to the 5' terminal side of the primary editing site.

Portion b) can also be a portion consisting of a nucleotide sequence having 90% or more identity with the nucleotide sequence of the region adjacent to the 3' terminal side of the secondary editing site. Here, the identity is preferably 95% or more, more preferably 97% or more, further more preferably 99% or more, even more preferably 99.5% or more, and especially preferably 99.9% or more.

In a preferred embodiment, portion b) consists of a nucleotide sequence complementary to the nucleotide sequence of the region adjacent to the 5' terminal side of the primary editing site, i.e., a nucleotide sequence identical to the nucleotide sequence of the region adjacent to the 3' terminal side of the secondary editing site.

The lengths of portions a) and b) are not particularly limited and can be set independently. The lengths of portions a) and b) can be, for example, 10 nucleotides or more, 20 nucleotides or more, 30 nucleotides or more, 35 nucleotides or more, 40 nucleotides or more, 45 nucleotides or more, 50 nucleotides or more, 60 nucleotides or more, 70 nucleotides or more, 80 nucleotides or more, 90 nucleotides or more, or 100 nucleotides or more, and can be 1000 nucleotides or less, 500 nucleotides or less, 300 nucleotides or less, 200 nucleotides or less, 190 nucleotides or less, 180 nucleotides or less, 170 nucleotides or less, 160 nucleotides or less, 150 nucleotides or less, 140 nucleotides or less, 130 nucleotides or less, 120 nucleotides or less, 110 nucleotides or less, 100 nucleotides or less, 90 nucleotides or less, 80 nucleotides or less, 70 nucleotides or less, 60 nucleotides or less, or 50 nucleotides or less, respectively.

The lengths of portions a) and b) can be, for example, 10 to 1000 nucleotides, 10 to 500 nucleotides, 10 to 300 nucleotides, 10 to 200 nucleotides, 10 to 100 nucleotides, 32 to 100 nucleotides, 35 to 70 nucleotides, or 35 to 50 nucleotides, respectively.

Portion x) is an optional portion consisting of a nucleotide sequence that is not identical to the nucleotide sequence of the secondary editing site and is determined by how the target site is to be modified. For example, when the intended modification is to replace one nucleotide pair or multiple contiguous nucleotide pairs in double-stranded genomic DNA, then portion x) consists of one nucleotide or multiple contiguous nucleotides intended to replace with the secondary editing site. When the intended modification is to delete one nucleotide pair or multiple contiguous nucleotide pairs in double-stranded genomic DNA, then portion x) is not included in the genome-editing polynucleotide. When the intended modification is to insert one nucleotide pair or multiple contiguous nucleotide pairs into double-stranded genomic DNA, then portion x) consists of one nucleotide or multiple contiguous nucleotides intended to be inserted into the secondary editing site.

Portion x) consists of a nucleotide sequence that is not identical to the nucleotide sequence of the secondary editing site. Portion x) can consist of any nucleotide sequence, as long as it is not an exact match to the nucleotide sequence of the secondary editing site, and can consist of, as an example, a nucleotide sequence with less than 90% identity to the nucleotide sequence of the secondary editing site. The identity is, for example, 50% or less, preferably 40% or less, more preferably 30% or less, further more preferably 20% or less, even more preferably 10% or less, and especially preferably 5% or less.

The length of portion x) is not particularly limited, and can be, for example, 2000 nucleotides or less, 1000 nucleotides or less, 900 nucleotides or less, 800 nucleotides or less, 700 nucleotides or less, 600 nucleotides or less, 500 nucleotides or less, 400 nucleotides or less, 300 nucleotides or less, 200 nucleotides or less, 100 nucleotides or less 90 nucleotides or less, 80 nucleotides or less, 70 nucleotides or less, 60 nucleotides or less, 50 nucleotides or less, 40 nucleotides or less, 30 nucleotides or less, 20 nucleotides or less, 10 nucleotides or less, 9 nucleotides or less, 8 nucleotides or less, 7 nucleotides or less, 6 nucleotides or less, 5 nucleotides or less, 4 nucleotides or less, 3 nucleotides or less, 2 nucleotides or less, or 1 nucleotide.

The genome-editing polynucleotide consists of portions a), x) and b), or portions a) and b), in order from the 5' terminal side. Thus, the length of the genome-editing polynucleotide can be determined by summing the lengths of each of the portions consisting of the genome-editing polynucleotide, that is, by summing the lengths of portion a), portion x), and portion b), or by summing the lengths of portions a) and b).

The genome-editing polynucleotide may contain one or more DNA high-affinity nucleotide analogs in one or both of portions a) and b). The original nucleotide can be replaced with a DNA high-affinity nucleotide analog so that the bases of the original nucleotide and the DNA high-affinity nucleotide analog are common, for example, when the original base is adenine, it can be replaced with a DNA high-affinity nucleotide analog containing adenine or an adenine analog. Multiple DNA high-affinity nucleotide analogs may be included adjacently, or they may be included intermittently without being adjacent. When included in both portions a) and b), the positions at which the DNA high-affinity nucleotide analogs are included can be chosen independently, and there's no requirement for the same number of them to be included in both portions.

A DNA high-affinity nucleotide analog is an artificial nucleic acid constituent unit with high binding affinity for DNA that can form polymer structures similar to natural nucleic acids. Examples of a DNA high-affinity nucleotide analog include a nucleotide with a cross-link between the oxygen atom at the 2' position and the carbon atom at the 4' position of the ribose ring, a peptide nucleic acid (PNA) with N-(2-aminoethyl)glycine instead of deoxyribose or ribose, and a morpholino nucleic acid with a morpholine ring instead of deoxyribose or ribose.

In an embodiment, the DNA high-affinity nucleotide analog is a nucleotide in which the oxygen atom at the 2' position and the carbon atom at the 4' position of the ribose ring are cross-linked, examples of which include LNA (Locked Nucleic Acid) in which the oxygen atom at the 2' position and the carbon atom at the 4' position are cross-linked through a methylene bridge, ENA in which they are cross-linked through an ethylene bridge, BNA (Bridged Nucleic Acid) ^{COC} in which they are cross-linked through -CH₂OCH₂-, BNA^{NC} in which they are cross-linked through -NR-CH₂- (R is methyl or a hydrogen atom), cMOE in which they are cross-linked through -CH₂(OCH₃)-, cEt in which they are cross-linked through -CH₂(CH₃)-, AmNA in which they are cross-linked through an amide bridge, and scpBNA in which they are cross-linked through a methylene bridge to form a cyclopropane at the 6' position.

The genome-editing polynucleotide may further contain a chemically modified nucleotide in addition to the DNA high-affinity nucleotide analog as its constituent unit. Examples of a chemically modified nucleotide include a nucleotide in which the phosphate group is replaced with a chemically modified phosphate group such as phosphorothioate (PS), methylphosphonate, and phosphorodithionate, etc., a nucleotide in which the hydroxyl group at the 2' position of the sugar (ribose) portion is replaced with -OR (R represents, for example, CH₃(2'-O-Me), CH₂CH₂OCH₃(2'-MOE), CH₂CH₂NHC(NH)NH₂, CH₂CONHCH₃, CH₂CH₂CN, etc.), a nucleotide in which a methyl group or cationic functional group is introduced at the 5-position of the pyrimidine base, and a nucleotide in which the carbonyl group at the 2-position of the pyrimidine base is replaced with a thiocarbonyl group. Furthermore, examples include a nucleotide in which the phosphate portion or hydroxyl portion is modified with, for example, biotin, an amino group, a lower alkylamine group, an acetyl group, etc., but are not limited to these nucleotides.

The genome-editing polynucleotide containing a DNA high-affinity nucleotide analog and optionally a chemically modified nucleotide can be produced by known synthetic methods.

The genome-editing polynucleotide in the present disclosure can modify genomic DNA in a cell as desired, similar to the single-stranded polynucleotide disclosed in Patent Literature 1 (Japanese Patent No. 6899564). In the modification, the genome-editing polynucleotide does not require the introduction of site-specific nucleases (ZFN proteins, TALEN proteins, Cas proteins, etc.), guide RNAs, or their expression vectors into the cell, as is the case with conventional genome-editing technologies.

Although not bound by theory, portion a) of the genome-editing polynucleotide hybridizes with the region adjacent to the 3' terminal side of the primary editing site set on one genomic DNA strand, while portion b) hybridizes with the region adjacent to the 5' terminal side of the primary editing site. As a result, when the genome-editing polynucleotide consists of portions a), x), and b), the portion x) protrudes, and when the genome-editing polynucleotide consists of portions a) and b), the primary editing site on the genomic DNA protrudes, without hybridizing to other sequences. This protruding segment is recognized by the repair system, allowing for modification of the target site on the double-stranded genomic DNA.

Specifically, when it is desired to insert one nucleotide pair or multiple contiguous nucleotide pairs in double-stranded genomic DNA, the primary editing site is set between two contiguous nucleotides where insertion is desired on one genomic DNA strand; portions a) and b) are set as described above; and portion x) is set at one nucleotide or multiple contiguous nucleotides that are desired to be inserted into the secondary editing site. By performing genome editing using the genome-editing polynucleotide consisting of portions a), x), and b) designed in this manner, a portion consisting of the nucleotide sequence complementary to portion x) can be inserted into the primary editing site, and portion x) can be inserted into the secondary editing site.

If it is desired to replace one nucleotide pair or multiple contiguous nucleotide pairs in double-stranded genomic DNA, the primary editing site is set at one nucleotide or multiple contiguous nucleotides where substitution is desired on one genomic DNA strand; portions a) and b) are set as described above; and portion x) is set at one nucleotide or multiple contiguous nucleotides that are desired to be replaced with the secondary editing site. By performing genome editing using the genome-editing polynucleotide consisting of portions a), x), and b) designed in this manner, a portion consisting of the nucleotide sequence complementary to portion x) can be inserted into the primary editing site, and portion x) can be inserted into the secondary editing site (shown in Fig. 8A).

If it is desired to delete one nucleotide pair or multiple contiguous nucleotide pairs in double-stranded genomic DNA, the primary editing site is set at one nucleotide or multiple contiguous nucleotides where deletion is desired on one genomic DNA strand; and portions a) and b) are set as described above. By performing genome editing using the genome-editing polynucleotide consisting of portions a) and b) designed in this manner, the primary editing site can be deleted from one genomic DNA strand and the secondary editing site can be deleted from the other genomic DNA strand, i.e., the target site can be deleted from the double-stranded genomic DNA.

Although not bound by theory, considering that there are many transcribed RNAs in the vicinity of genomic DNA, for example, when the target site is in a genomic DNA region that is transcribed into RNA and the primary editing site is set on the genomic DNA sense strand, the genome-editing efficiency is suggested to be not good. This is because the genome-editing polynucleotide may bind, rather than to the region adjacent to the 3' terminal side of the primary editing site and the region adjacent to the 5' terminal side of the primary editing site on the genomic DNA sense strand, preferentially to the region with the same nucleotide sequence as the above two regions in the surrounding RNAs. When genome-editing efficiency is a priority, it is preferable to set the primary editing site on the antisense strand rather than on the sense strand of genomic DNA.

As described above, portion a) of the genome-editing polynucleotide can hybridize with the region adjacent to the 3' terminal side of the primary editing site set on one genomic DNA strand, and portion b) of the genome-editing polynucleotide can hybridize with the region adjacent to the 5' terminal side of the primary editing site, thereby modifying the target site on the double-stranded genomic DNA. Therefore, the genome-editing polynucleotide may have any nucleotide sequence added to one or both ends, i.e., to the 5' terminal of portion a) and/or to the 3' terminal of portion b), as long as the addition of the sequence does not affect hybridization of portion a) to the region adjacent to the 3' terminal side of the primary editing site, and portion b) to the 5' terminal side of the primary editing site. The genome-editing polynucleotide in the present disclosure encompasses those to which such an optional nucleotide sequence is added.

For the genome-editing polynucleotide with an optional nucleotide sequence added at its terminal, calculation of the identity with each of the regions adjacent to the 5' terminal side and the 3' terminal side of the secondary editing site is performed between the nucleotide sequence of portion a) and the nucleotide sequence of the region adjacent to the 5' terminal side of the secondary editing site, and between the nucleotide sequence of portion b) and the nucleotide sequence of the region adjacent to the 3' terminal side of the secondary editing site. The added optional nucleotide sequence is considered to be absent in the identity calculation.

### Promoting polynucleotide for genome editing

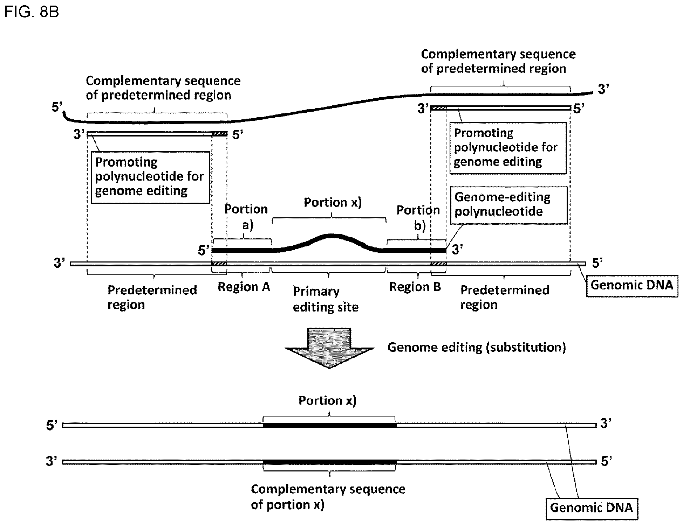

The genome-editing polynucleotide can be used in combination with a promoting polynucleotide for genome editing to improve the genome-editing efficiency. The promoting polynucleotide for genome editing is a polynucleotide that is capable of hybridizing to a nucleic acid sequence that is complementary to a predetermined region (also referred to as a complementary sequence of the predetermined region) on a genomic DNA strand on which the primary editing site is set. The details of the promoting polynucleotide for genome editing are described below (see also Fig. 8B).

The predetermined region is set to overlap at its 5' terminal part with region A or at its 3' terminal part with region B. On the genomic DNA strand where the primary editing site is set, a region at a position corresponding to portion a) of the genome-editing polynucleotide is defined as region A, and a region at a position corresponding to portion b) of the genome-editing polynucleotide is defined as region B.

The length of overlap at the 5' terminal part of the predetermined region with region A is not limited as long as it is the same as or shorter than that of region A. The length of overlap at the 3' terminal part of the predetermined region with region B is not limited as long as it is the same as or shorter than that of region B. The length of these overlaps can be set independently, and can be, for example, 1 nucleotide or more, 2 nucleotides or more, 3 nucleotides or more, 4 nucleotides or more, 5 nucleotides or more, 6 nucleotides or more, 7 nucleotides or more, 8 nucleotides or more, 9 nucleotides or more, or 10 nucleotides or more, and can be 20 nucleotides or less, 19 nucleotides or less, 18 nucleotides or less, 17 nucleotides or less, 16 nucleotides or less, 15 nucleotides or less, 14 nucleotides or less, 13 nucleotides or less, 12 nucleotides or less, or 11 nucleotides or less. The length of these overlaps can also be independently, for example, 1 to 20 nucleotides, 5 to 20 nucleotides, 5 to 15 nucleotides, 8 to 12 nucleotides, or 9 to 11 nucleotides.

The complementary sequence of the predetermined region is a region on another polynucleotide that is complementary to the predetermined region on the genomic DNA strand. The complementary sequence of the predetermined region is typically found in nature in RNAs transcribed from the genomic DNA strand on which the predetermined region is set.

The promoting polynucleotide for genome editing can hybridize to the complementary sequence of the predetermined region on the genomic DNA strand on which the primary editing site is set.

The promoting polynucleotide for genome editing may be a polynucleotide consisting of a nucleotide sequence having 90% or more identity with the nucleotide sequence of the predetermined region. Here, the identity is preferably 95% or more, more preferably 97% or more, further more preferably 99% or more, even more preferably 99.5% or more, and especially preferably 99.9% or more.

In a preferred embodiment, the promoting polynucleotide for genome editing consists of a nucleotide sequence identical to the nucleotide sequence of the predetermined region described above.

The promoting polynucleotide for genome editing includes two types of promoting polynucleotides: a promoting polynucleotide for genome editing in which the predetermined region is set to overlap at its 5' terminal part with region A, and a promoting polynucleotide for genome editing in which the predetermined region is set to overlap at its 3' terminal part with region B. The genome-editing efficiency can be improved by using one of these two types of promoting polynucleotides for genome editing in combination with the genome-editing polynucleotide. The genome-editing efficiency can be further improved by using both of these two types of promoting polynucleotides for genome editing in combination with the genome-editing polynucleotide.

In an embodiment using the above two types of promoting polynucleotides for genome editing, the ratio of the two types of promoting polynucleotides for genome editing used is not limited, and the molar concentrations of the two may be comparable, or the molar concentration of one may be much higher than that of the other.

The length of the promoting polynucleotide for genome editing is the same as or longer than the length of the overlap with region A, when the predetermined region is set to overlap at its 5' terminal part with region A. The length of the promoting polynucleotide for genome editing is the same as or longer than the length of the overlap with region B, when the predetermined region is set to overlap at its 3' terminal part with region B. When the two types of promoting polynucleotides for genome editing are used, the length of each promoting polynucleotide for genome editing does not have to be the same and can be set independently.

The length of the promoting polynucleotide for genome editing can independently be, for example, 20 nucleotides or more, 30 nucleotides or more, 40 nucleotides or more, 50 nucleotides or more, 60 nucleotides or more, 70 nucleotides or more, 80 nucleotides or more, 90 nucleotides or more, 100 nucleotides or more, 110 nucleotides or more, 120 nucleotides or more, or 130 nucleotides or more, and can be 1000 nucleotides or less, 500 nucleotides or less, 300 nucleotides or less, 250 nucleotides or less, 200 nucleotides or less, 190 nucleotides or less, 180 nucleotides or less, 170 nucleotides or less, 160 nucleotides or less, 150 nucleotides or less, 140 nucleotides or less or 130 nucleotides or less.

The length of the promoting polynucleotide for genome editing can independently be, for example, 10 to 1000 nucleotides, 20 to 500 nucleotides, 30 to 300 nucleotides, 40 to 200 nucleotides, 50 to 200 nucleotides, 60 to 200 nucleotides, 70 to 150 nucleotides, or 70 to 130 nucleotides.

Although not bound by theory, in an environment where there are many other polynucleotides that have regions complementary to the primary editing site or its neighboring regions (namely region A and region B described above), the binding between the genome-editing polynucleotide and the genomic DNA strand on which the primary editing site is set may be competitively inhibited by these other polynucleotides. Consequently, the genome-editing efficiency by the genome-editing polynucleotide is thought to be reduced.

For example, when the target site is located in a genomic DNA region that is transcribed into RNA and the primary editing site is set on the genomic DNA antisense strand, the genome-editing polynucleotide may compete with the surrounding RNA for binding to the genomic DNA antisense strand. This competition may inhibit the binding between the genome-editing polynucleotide and the genomic DNA antisense strand. Consequently, the genome-editing efficiency is thought to be lower than in cases where no surrounding RNA is present.

In such an environment, when the genome-editing polynucleotide coexists with the promoting polynucleotide for genome editing, the aforementioned other polynucleotides (e.g., RNA) can bind to the promoting polynucleotide for genome editing. Consequently, the inhibition of binding between the genome-editing polynucleotide and the genomic DNA strand (e.g., antisense strand) on which the primary editing site is set can be alleviated, and the genome-editing efficiency is expected to improve (Fig. 8B).

In an embodiment in which the target site is located in a genomic DNA region that is transcribed into RNA and the primary editing site is set on the genomic DNA antisense strand, it is preferable for the promoting polynucleotide for genome editing to have a high binding affinity for the RNA. The promoting polynucleotide for genome editing may contain one or more RNA high-affinity nucleotide analogs. The original nucleotide can be replaced with an RNA high-affinity nucleotide analog so that the bases of the original nucleotide and the RNA high-affinity nucleotide analog are common, for example, when the original base is adenine, it can be replaced with an RNA high-affinity nucleotide analog containing adenine or an adenine analog. Multiple RNA high-affinity nucleotide analogs may be included adjacently, or they may be included intermittently without being adjacent. When the two types of promoting polynucleotides for genome editing are used, each promoting polynucleotide for genome editing can contain an RNA high affinity nucleotide analog at a position selected independently of each other. The number of the nucleotide analogs does not have to be the same.

Although not bound by theory, in an embodiment in which the target site is located in a genomic DNA region that is transcribed into RNA and the primary editing site is set on the genomic DNA antisense strand, when the target site is set in a region that is transcribed into an intron part of mRNA precursor, there would be more intense competition between the genome-editing polynucleotide and RNA for binding to the genomic DNA antisense strand, and the genome-editing efficiency is thought to be lower compared to when the target site is set in a region that is transcribed into an exon part of mRNA precursor. This is because a large number of intron parts, excised by splicing from the mRNA precursor, are present in the vicinity of the genomic DNA.

Therefore, the promoting polynucleotide for genome editing can improve the genome-editing efficiency both when the target site is located in a genomic DNA region that is transcribed into an intron part of mRNA precursor and the primary editing site is set on the genomic DNA antisense strand, and when the target site is located in a genomic DNA region that is transcribed into an exon part of mRNA precursor and the primary editing site is set on the genomic DNA antisense strand. In particular, when the target site is located in a genomic DNA region that is transcribed into an intron part of mRNA precursor and the primary editing site is set on the genomic DNA antisense strand, the promoting polynucleotide for genome editing can exhibit a strong effect on improvement in genome-editing efficiency.

In an embodiment in which the target site is set on a double-stranded genomic DNA region that is transcribed into RNA and the primary editing site is set on the genomic DNA antisense strand, the genome-editing polynucleotide is also referred to as genome-editing sense polynucleotide, and the promoting polynucleotide for genome editing is also referred to as the promoting antisense polynucleotide for genome editing.

### Kit for genome editing

The present disclosure provides, in one aspect, a kit for genome editing that contains the aforementioned genome-editing polynucleotide and the aforementioned promoting polynucleotide for genome editing. The kit for genome editing preferably contains two types of the promoting polynucleotides for genome editing: the promoting polynucleotide for genome editing in which the predetermined region is set to overlap at its 5' terminal part with region A, and the promoting polynucleotide for genome editing in which the predetermined region is set to overlap at its 3' terminal part with region B.

The kit for genome editing can contain, instead of the genome-editing polynucleotide, an expression vector of the genome-editing polynucleotide, or a conjugate of the genome-editing polynucleotide or the expression vector of the genome-editing polynucleotide with one or more other substances. The kit for genome editing can contain, instead of the promoting polynucleotide for genome editing, an expression vector of the promoting polynucleotide for genome editing, or a conjugate of the promoting polynucleotide for genome editing or the expression vector of the promoting polynucleotide for genome editing with one or more other substances.

Other substances conjugated to the genome-editing polynucleotide are not limited as long as they can form a conjugate with the genome-editing polynucleotide and do not interfere with the function of the genome-editing polynucleotide, i.e., the function of modifying the target site on genomic DNA. Other substances conjugated to the promoting polynucleotide for genome editing are not limited as long as they can form a conjugate with the promoting polynucleotide for genome editing and do not interfere with the function of the promoting polynucleotide for genome editing, i.e., the function of improving genome-editing efficiency by the genome-editing polynucleotide.

Examples of other substances conjugated to the genome-editing polynucleotide and the promoting polynucleotide for genome editing include functional peptides such as nuclear transfer signals and membrane-permeable peptides; hydrophilic polymers such as polyethylene glycol and dextran; and labeling substances such as radioisotopes, fluorescein and its derivatives, rhodamine and its derivatives, fluorescent proteins, phycobiliproteins, biotin, and avidin.

The kit for genome editing can further contain a reagent including a nucleic acid transfer reagent such as a transfection reagent, and a buffer solution.

The components of the kit for genome editing, such as the aforementioned genome-editing polynucleotide, its expression vector or its conjugate, the aforementioned promoting polynucleotide for genome editing, its expression vector or its conjugate, each of the reagents, may be contained in the kit for genome editing in separate forms, or may be contained in the kit for genome editing in the form of a composition in which multiple components are combined.

The kit for genome editing may further contain, for example, an instrument to be used in genome editing, instructions for use.

In an embodiment, the kit for genome editing is a kit for genome editing for the treatment or prevention of a disease, and can contain, in addition to a therapeutically or prophylactically effective amount of the genome-editing polynucleotide, its expression vector, or a conjugate of the genome-editing polynucleotide or its expression vector with one or more other substances, other pharmaceutically acceptable components, such as a pharmaceutically acceptable additive (buffer, stabilizer, preservative, excipient, etc.), and a pharmaceutically acceptable medium (water, saline, phosphate buffered saline (PBS), etc.). The kit for genome editing for the treatment or prevention of a disease can be used to treat or prevent a disease caused by an abnormality in genomic DNA.

### Method for genome editing and method for producing genome-edited cell or organism

The present disclosure provides, in one aspect, a method for modifying a target site on double-stranded genomic DNA of a cell or an organism (also referred to as a "method for genome editing" in the present disclosure), including the step of treating the cell or the organism using the aforementioned kit for genome editing. The present disclosure also provides, in one aspect, a method for producing a cell or an organism in which a target site on double-stranded genomic DNA has been modified (also referred to as a "method for producing a genome-edited cell or organism" in the present disclosure), including the step of treating the cell or the organism using the aforementioned kit for genome editing.

The cell to be treated using the kit for genome editing is a cell whose genomic DNA is desired to be modified and can originate from a variety of organisms, including the prokaryotes and eukaryotes mentioned above. If the cell is a cell of a multicellular organism, the cell can be derived from various tissues of the organism. The cell may be a somatic cell or a germ cell. The cell may be a differentiated or undifferentiated cell, a tissue stem cell, an embryonic stem cell, or an induced pluripotent stem cell (iPS cell).

The cell to be treated using the kit for genome editing may be in the form of a single cell or a cell population containing one or more types of cells.

The organism to be treated using the kit for genome editing is an organism whose genomic DNA is desired to be modified and can be a variety of organisms, including the prokaryotes and eukaryotes mentioned above. The organism may be a mature individual, an embryo or fetus in the developmental stage, or in the case of plants, a seed, seedling, or bulb.

In an embodiment, the cell can be represented as a cell excluding human gametes and fertilized eggs.

In an embodiment, the organism excludes humans and can be represented as a non-human organism. In another embodiment, the organism excludes humans and animals and can be represented as a non-human, non-animal organism.

Further, in an embodiment, the cell excludes human gametes and fertilized eggs, and the organism excludes humans. In another preferred embodiment, the cell excludes human gametes and fertilized eggs, and the organism excludes humans and animals.

Treatment of the cell or organism using the kit for genome editing means making the genome-editing polynucleotide, its expression vector, or a conjugate of the genome-editing polynucleotide or its expression vector with one or more other substances, as well as the promoting polynucleotide for genome editing, its expression vector, or a conjugate of the promoting polynucleotide for genome editing or its expression vector with one or more other substances contained in the kit for genome editing, coexist with the cell, or administering them to the organism. By this coexistence with the cell or administration to the organism, the genome-editing polynucleotide, its expression vector, or a conjugate of the genome-editing polynucleotide or its expression vector with one or more other substances and the promoting polynucleotide for genome editing, its expression vector, or a conjugate of the promoting polynucleotide for genome editing or its expression vector with one or more other substances are introduced into the cell or organism, thereby the genomic DNA in the cell or organism can be edited.

Treatment of the cell or organism using the kit for genome editing can be performed by various known methods selected by those skilled in the art in consideration of factors such as the type or nature of the cell or organism. Treatment of the cell or organism can include, for example, microinjection, electroporation, DEAE-dextran treatment, transfection using a transfection reagent (jetPEI (PolyPlus-transfection), Lipofectamine, etc.), transfection using a lipid nanoparticle, and the hydrodynamic method.

In an embodiment, the treatment of a human cell is performed in vitro or ex vivo.

In general, genome editing may increase or decrease the characteristic of a cell or organism, such as nutrient requirements, sensitivity or resistance to chemicals, may confer a characteristic not present in the cell or organism before editing, or may cause the cell or organism to lose a characteristic it had before editing. The desired genome-edited cell or organism can be selected using such a change in characteristic as an indicator. For example, if genome editing is expected to increase or confer drug resistance, the genome-edited cell or organism can be selected by culturing cells treated using the kit for genome editing with an appropriate medium containing the drug, or raising organisms treated using the kit for genome editing with an appropriate feed containing the drug. The method of selection and evaluation of the characteristic of the cells or organisms, and the culture or rearing conditions that enable selection of the cells or organisms can be determined as appropriate within the ordinary practicing capability of those skilled in the art.

The method for genome editing and the method for producing a genome-edited cell or organism described above can include, in addition to the step of treating the cell or organism using the kit for genome editing, the step of selecting a cell or organism whose genomic DNA has been edited using a characteristic of the cell or organism that is changed by the genome-editing polynucleotide as an indicator.

### Further invention provided by the present disclosure

According to the present disclosure, the following inventions are also provided. Examples of correspondence between terms used in the following inventions and those used in the aforementioned inventions are shown in Table 1.

**[Table 1]**

| TERMS USED IN THE FOLLOWING INVENTIONS | TERMS USED IN THE AFOREMENTIONED INVENTIONS |
|---|---|
| target region | target site |
| target region that matches the antisense strand of genomic region | primary editing site set on genomic DNA antisense strand |
| sequence adjacent upstream of target region | region adjacent to 3' terminal side of primary editing site |
| sequence adjacent downstream of target region | region adjacent to 5' terminal side of primary editing site |
| upstream homology arm | a) portion capable of hybridizing to region adjacent to 3' terminal side of primary editing site |
| downstream homology arm | b) portion capable of hybridizing to region adjacent to 5' terminal side of primary editing site |
| optional sequence between upstream homology arm and downstream homology arm | x) optional portion consisting of nucleotide sequence that is not identical to nucleotide sequence of secondary editing site, and portion consisting of nucleotide sequence that is identical to nucleotide sequence of secondary editing site |
| other single-stranded polynucleotide | single-stranded promoting polynucleotide for genome editing |
| Single-stranded antisense polynucleotide | single-stranded promoting antisense polynucleotide for genome editing |
| having sequence capable of hybridizing to region having sequence that matches terminal part of genome-editing polynucleotide | being capable of hybridizing to nucleic acid sequence that is complementary to predetermined region on genomic DNA strand in which primary editing site is set, wherein said predetermined region overlaps at its 5' terminal part with region A or overlaps at its 3' terminal part with region B, in which region on said genomic DNA strand at position corresponding to portion a) of genome-editing polynucleotide is defined as region A, and region on said genomic DNA strand at position corresponding to portion b) of genome-editing polynucleotide is defined as region B |
| having sequence capable of hybridizing to region having sequence that matches 5' terminal part of genome-editing polynucleotide | being capable of hybridizing to nucleic acid sequence that is complementary to predetermined region on genomic DNA strand in which primary editing site is set, wherein said predetermined region overlaps at its 5' terminal part with region A, in which region on said genomic DNA strand at position corresponding to portion a) of genome-editing polynucleotide is defined as region A |
| having sequence capable of hybridizing to region having sequence that matches 3' terminal part of genome-editing polynucleotide | being capable of hybridizing to nucleic acid sequence that is complementary to predetermined region on genomic DNA strand in which primary editing site is set, wherein said predetermined region overlaps at its 3' terminal part with region B, in which region on said genomic DNA strand at position corresponding to portion b) of genome-editing polynucleotide is defined as region B |

(1) A method for editing a genome in a eukaryotic cell, including: the step (A) of causing a single-stranded genome-editing polynucleotide to interact with a genome in the cell in a sequence-specific manner,
   wherein the genome-editing polynucleotide is capable of hybridizing to a sequence adjacent upstream and a sequence adjacent downstream of a target region on the genome (e.g., a target region that matches the antisense strand of a genomic region where RNA is transcribed),
   the single-stranded genome-editing polynucleotide (e.g., a genome-editing sense polynucleotide) has an upstream homology arm and a downstream homology arm,
   the upstream homology arm has a sequence capable of hybridizing to a sequence adjacent upstream of the target region,
   the downstream homology arm has a sequence capable of hybridizing to a sequence adjacent downstream of the target region,
   the genome-editing polynucleotide can hybridize, through the upstream homology arm and the downstream homology arm, to at least the upstream and downstream of the target region,
   the genome-editing polynucleotide may have an optional sequence between the upstream homology arm and the downstream homology arm, or may have no sequence between them,
   the step (A) is performed in the presence of other single-stranded polynucleotide (e.g., a single-stranded antisense polynucleotide),
   the other polynucleotide (e.g., the single-stranded antisense polynucleotide) has a sequence capable of hybridizing to a region having a sequence that matches the terminal part of the genome-editing polynucleotide (the region is, for example, a partial region of RNA transcribed from the genomic region and having a nucleotide sequence that matches the terminal part of the genome-editing sense polynucleotide) (the sequence that matches the terminal part of the genome-editing polynucleotide is, for example, a sequence that is complementary to the partial region),
   the length of the other polynucleotide (e.g., the single-stranded antisense polynucleotide) is 40 nucleotides or more, and
   through the step, the target region is replaced with the sequence between the upstream homology arm and the downstream homology arm of the single-stranded genome-editing polynucleotide (e.g., the single-stranded genome-editing sense polynucleotide), provided that, when there is no sequence between the upstream homology arm and the downstream homology arm of the single-stranded genome-editing polynucleotide, the target region is deleted.
(2) The method according to (1) above, wherein the single-stranded genome-editing polynucleotide is a polynucleotide selected from the group consisting of DNA, RNA, and a combination thereof.
(3) The method according to (1) or (2) above, wherein the other single-stranded polynucleotide is DNA.
(4) The method according to any of (1) to (3) above, wherein the region having a sequence that matches the terminal part has a length of 5 bases or more.
(5) The method according to any of (1) to (4) above, wherein the region having a sequence that matches the terminal part has a length of 20 bases or less.
(6) The method according to any of (1) to (5) above, wherein the single-stranded genome-editing polynucleotide has a length of 60 to 200 bases.
(7) The method according to any of (1) to (6) above, wherein the other single-stranded polynucleotide has a length of 50 to 200 bases.
(8) The method according to any of (1) to (7) above, wherein the target region is in an intron.
(9) The method according to any of (1) to (8) above, wherein the step (A) is performed in the presence of at least two types of the other single-stranded polynucleotides capable of hybridizing, at least one type of the other single-stranded polynucleotide (a first other polynucleotide) among them has a sequence capable of hybridizing to a region having a sequence that matches the 5' terminal part of the genome-editing polynucleotide (the sequence that matches the 5' terminal part of the genome-editing polynucleotide is, for example, a sequence that is complementary to the region), and at least one type of the other single-stranded polynucleotide (a second other polynucleotide) among them has a sequence capable of hybridizing to a region having a sequence that matches the 3' terminal part of the genome-editing polynucleotide (the sequence that matches the 3' terminal part of the genome-editing polynucleotide is, for example, a sequence that is complementary to the region).
(10) A kit for genome editing for use in the method according to any of (1) to (8) above, containing a combination of the single-stranded genome-editing polynucleotide and the other single-stranded polynucleotide.
(11) A kit for genome editing for use in the method according to any of (1) to (8) above, containing DNA encoding the single-stranded genome-editing polynucleotide and DNA encoding the other single-stranded polynucleotide, wherein the DNAs are contained in an expressible manner in one or more expression vectors.
(12) A kit for genome editing for use in the method according to any of (1) to (8) above, containing DNA encoding the single-stranded genome-editing polynucleotide and the other single-stranded polynucleotide, wherein the DNA encoding the single-stranded genome-editing polynucleotide is contained in an expressible manner in an expression vector, and the other single-stranded polynucleotide is DNA.
(13) A kit for genome editing for use in the method according to any of (1) to (8) above, containing a polynucleotide encoding the single-stranded genome-editing polynucleotide and DNA encoding the other single-stranded polynucleotide, wherein the DNAs are contained in an expressible manner in one or more expression vectors.
(14) A kit for genome editing for use in the method according to (9) above, containing a combination of the single-stranded genome-editing polynucleotide and the at least two types of the other single-stranded polynucleotides.
(15) A kit for genome editing for use in the method according to (9) above, containing DNA encoding the single-stranded genome-editing polynucleotide and DNAs encoding the at least two types of the other single-stranded polynucleotides, wherein the DNAs are contained in an expressible manner in one or more expression vectors.
(16) A kit for genome editing for use in the method according to (9) above, containing DNA encoding the single-stranded genome-editing polynucleotide and the at least two types of the other single-stranded polynucleotides, wherein the DNA encoding the single-stranded genome-editing polynucleotide is contained in an expressible manner in an expression vector, and the other single-stranded polynucleotides are DNAs.
(17) A kit for genome editing for use in the method according to (9) above, containing a polynucleotide encoding the single-stranded genome-editing polynucleotide and DNAs encoding the at least two types of the other single-stranded polynucleotides, wherein the DNAs are contained in an expressible manner in one or more expression vectors.

(1A) A method for editing a genome in a eukaryotic cell, including: the step (A) of causing a single-stranded genome-editing sense polynucleotide to interact with a genome in the cell in a sequence-specific manner,
   wherein the genome-editing sense polynucleotide is capable of hybridizing to a sequence adjacent upstream and a sequence adjacent downstream of a target region that matches the antisense strand of a genomic region where RNA is transcribed,
   the single-stranded genome-editing sense polynucleotide has an upstream homology arm and a downstream homology arm,
   the upstream homology arm has a sequence capable of hybridizing to a sequence adjacent upstream of the target region,
   the downstream homology arm has a sequence capable of hybridizing to a sequence adjacent downstream of the target region,
   the genome-editing sense polynucleotide can hybridize, through the upstream homology arm and the downstream homology arm, to at least the upstream and downstream of the target region,
   the genome-editing sense polynucleotide may have an optional sequence between the upstream homology arm and the downstream homology arm, or may have no sequence between them,
   the step (A) is performed in the presence of a single-stranded antisense polynucleotide,
   the antisense polynucleotide has a sequence capable of hybridizing to a region which is a partial region of RNA transcribed from the genomic region and which has a sequence that matches the terminal part of the genome-editing sense strand polynucleotide (the sequence that matches the terminal part of the genome-editing sense polynucleotide is, for example, a sequence that is complementary to the partial region),
   the length of the antisense polynucleotide is 40 nucleotides or more, and
   through the step, the target region is replaced with the sequence between the upstream homology arm and the downstream homology arm of the single-stranded genome-editing sense polynucleotide, provided that, when there is no sequence between the upstream homology arm and the downstream homology arm of the single-stranded genome-editing sense polynucleotide, the target region is deleted.
(2A) The method according to (1A) above, wherein the single-stranded genome-editing sense polynucleotide is a polynucleotide selected from the group consisting of DNA, RNA, and a combination thereof.
(3A) The method according to (1A) or (2A) above, wherein the single-stranded antisense polynucleotide is DNA.
(4A) The method according to any of (1A) to (3A) above, wherein the terminal part has a length of 5 bases or more.
(5A) The method according to any of (1A) to (4A) above, wherein the terminal part has a length of 20 bases or less.
(6A) The method according to any of (1A) to (5A) above, wherein the single-stranded genome-editing sense polynucleotide has a length of 60 to 200 bases.
(7A) The method according to any of (1A) to (6A) above, wherein the single-stranded antisense polynucleotide has a length of 50 to 200 bases.
(8A) The method according to any of (1A) to (7A) above, wherein the target region is in an intron.
(9A) The method according to any of (1A) to (8A) above, wherein the step (A) is performed in the presence of at least two types of the single-stranded antisense polynucleotides capable of hybridizing, at least one type of the antisense polynucleotide among them has a sequence capable of hybridizing to a region which is a partial region of RNA transcribed from the genomic region and which has a sequence that matches the 5' terminal part of the genome-editing sense strand polynucleotide (the sequence that matches the 5' terminal part of the genome-editing sense polynucleotide is, for example, a sequence that is complementary to the region), and at least one type of the antisense polynucleotide among them has a sequence capable of hybridizing to a region which is a partial region of RNA transcribed from the genomic region and which has a sequence that matches the 3' terminal part of the genome-editing sense strand polynucleotide (the sequence that matches the 3' terminal part of the genome-editing sense polynucleotide is, for example, a sequence that is complementary to the region).
(10A) A kit for genome editing for use in the method according to any of (1A) to (8A) above, containing a combination of the single-stranded genome-editing sense polynucleotide and the single-stranded antisense polynucleotide.
(11A) A kit for genome editing for use in the method according to any of (1A) to (8A) above, containing DNA encoding the single-stranded genome-editing sense polynucleotide and DNA encoding the single-stranded antisense polynucleotide, wherein the DNAs are contained in an expressible manner in one or more expression vectors.
(12A) A kit for genome editing for use in the method according to any of (1A) to (8A) above, containing DNA encoding the single-stranded genome-editing sense polynucleotide and the single-stranded antisense polynucleotide, wherein the DNA encoding the single-stranded genome-editing sense polynucleotide is contained in an expressible manner in an expression vector, and the single-stranded antisense polynucleotide is DNA.
(13A) A kit for genome editing for use in the method according to any of (1A) to (8A) above, containing a polynucleotide encoding the single-stranded genome-editing sense polynucleotide and DNA encoding the single-stranded antisense polynucleotide, wherein the DNAs are contained in an expressible manner in one or more expression vectors.
(14A) A kit for genome editing for use in the method according to (9A) above, containing a combination of the single-stranded genome-editing sense polynucleotide and the at least two types of the single-stranded antisense polynucleotides.
(15A) A kit for genome editing for use in the method according to (9A) above, containing DNA encoding the single-stranded genome-editing sense polynucleotide and DNAs encoding the at least two types of the single-stranded antisense polynucleotides, wherein the DNAs are contained in an expressible manner in one or more expression vectors.
(16A) A kit for genome editing for use in the method according to (9A) above, containing DNA encoding the single-stranded genome-editing sense polynucleotide and the at least two types of the single-stranded antisense polynucleotides, wherein the DNA encoding the single-stranded genome-editing sense polynucleotide is contained in an expressible manner in an expression vector, and the single-stranded antisense polynucleotides are DNAs.
(17A) A kit for genome editing for use in the method according to (9A) above, containing a polynucleotide encoding the single-stranded genome-editing sense polynucleotide and DNAs encoding the at least two types of the single-stranded antisense polynucleotides, wherein the DNAs are contained in an expressible manner in one or more expression vectors.

The term "sequence that match" in the item "Further invention provided by the present disclosure" means a sequence (especially a nucleotide sequence) at a location that matches the sequence. For example, DNA and RNA transcribed from the DNA have matched sequences. The correspondence between DNA and RNA sequences is known to be A, T, G, and C in DNA are A, U, G, and C, respectively. The term "complementary" means that two polynucleotides are capable of hybridizing to each other by the Watson-Crick type binding in the intracellular or in vitro environment comparable thereto. Generally, A and T (or U) have complementarity to each other, and G and C have complementarity to each other. The "complementarity" in the term " having complementarity" can be 90% or more, 95% or more, or full complementarity (i.e., 100% complementarity).

The term "target region" refers to a region targeted by a genome modification system.

### <Method for editing genome and method for producing cell having edited genome>

According to the present invention, a method for editing a genome is provided. According to the present invention, a method for targeting a target region of a genome is provided. According to the present invention, a method for obtaining a cell having an edited genome is provided.

In all embodiments, a cell can be a eukaryotic cell. In all embodiments, a cell can preferably be a cloned cell. In all embodiments, the methods according to the present invention can be in vitro methods. Thus, in the methods according to the present invention, an isolated cell can be obtained. An isolated cell obtained in the present invention can be a cell in which a nucleotide sequence to be inserted is inserted in the target sequence.

A cell obtained in the present invention may or may not subsequently be cloned. In a preferred aspect, a cell obtained in the present invention may be cloned and obtained as a cloned cell. Uniformity of the genome has the advantage of facilitating subsequent industrial applications or genetic manipulation, etc.

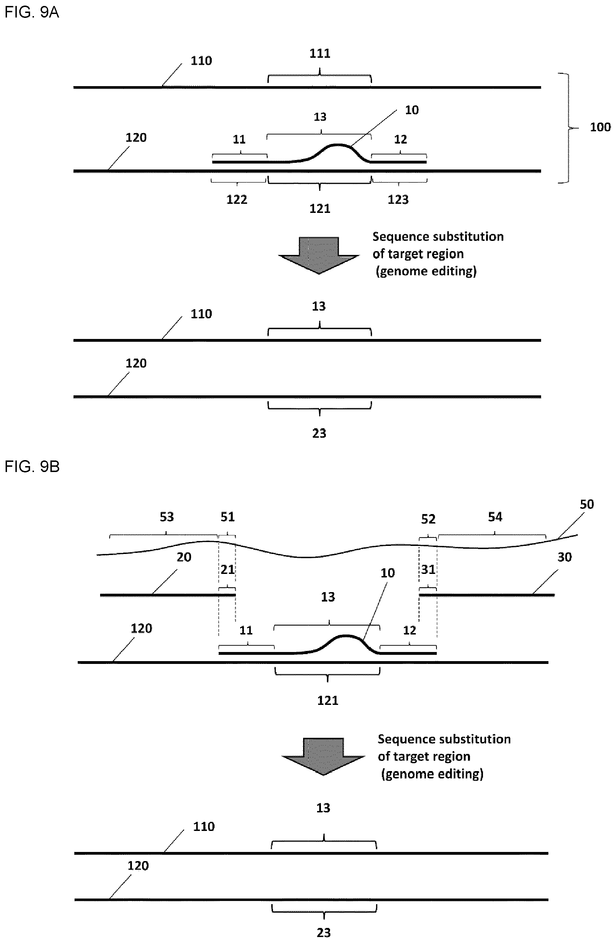

In the following, the method for editing a genome (or for producing a cell having an edited genome) provided by the present invention will be described using Fig. 9A and Fig.9B. The method described in Fig. 9A is disclosed in detail in WO 2018/030536 and JP 2016-220639 A. A region (100) of a genome where RNA is transcribed consists of a sense strand (110) and an antisense strand (120).

According to the present invention, there is provided a method for editing a genome (or a method for producing a cell having an edited genome), including:
the step (A) of causing a single-stranded genome-editing sense polynucleotide (10) to interact with a genome (100) in a sequence-specific manner, wherein the genome-editing sense polynucleotide (10) is capable of hybridizing to a sequence (122) adjacent upstream and a sequence (123) adjacent downstream of a target region (121) that matches the antisense strand of a genomic region where RNA is transcribed). Herein, "adjacent" means existing next to (i.e., contiguous in sequence).

According to the present invention, there is provided a method for editing a genome in a cell, including:
the step (A) of causing a single-stranded genome-editing sense polynucleotide (10) to interact with a genome in the cell in a sequence-specific manner, wherein the genome-editing sense polynucleotide (10) is capable of hybridizing to a sequence (122) adjacent upstream and a sequence (123) adjacent downstream of a target region (121) that matches the antisense strand of a genomic region where RNA is transcribed).

According to the present invention, there is provided a method for targeting a genome in a cell, including:
the step (A) of causing a single-stranded genome-editing sense polynucleotide (10) to interact with a genome (100) in the cell in a sequence-specific manner, wherein the genome-editing sense polynucleotide (10) is capable of hybridizing to a sequence (122) adjacent upstream and a sequence (123) adjacent downstream of a target region (121) that matches the antisense strand of a genomic region where RNA is transcribed).

In an aspect of the present invention, the single-stranded genome-editing sense polynucleotide (10) has an upstream homology arm (11) and a downstream homology arm (12). The upstream homology arm (11) has a sequence capable of hybridizing to a sequence (122) adjacent upstream of the target region, and the downstream homology arm (12) has a sequence capable of hybridizing to a sequence (123) adjacent downstream of the target region (121). The single-stranded genome-editing sense polynucleotide (10) can hybridize, through the upstream homology arm (11) and the downstream homology arm (12), to the sequence (122) adjacent upstream and the sequence (123) adjacent downstream of the target region, respectively.

The single-stranded genome-editing sense polynucleotide (10) can have an optional sequence (13) between the upstream homology arm (11) and the downstream homology arm (12). For example, the optional sequence (13) is replaced with a sequence (111) of a region that matches the complementary strand of the target region (121). Specifically, the target region (121) is replaced with the complementary sequence (23) of the optional sequence (13), and the region (111) that matches the target sequence on the complementary strand is replaced with the sequence of the optional sequence (13). Therefore, the optional sequence (13) is determined by how the region (111) that matches the target sequence on the complementary strand is to be modified. That is, the region (111) can be replaced with the optional sequence (13). For example, a part of or the whole region (111) that matches the target sequence on the complementary strand can be replaced with a new sequence (i.e., the sequence of the optional sequence (13)). When the sequence of the optional sequence (13) has a sequence insertion relative to the region (111) that matches the target sequence on the complementary strand, the new sequence can be inserted into the region (111). When the sequence of the optional sequence (13) has a deletion of part or all of the sequence relative to the region (111) that matches the target sequence on the complementary strand, the sequence can be deleted from the region (111). The single-stranded genome-editing sense polynucleotide (10) may not have a base in the optional sequence (13) {i.e., the upstream homology arm (11) and the downstream homology arm (12) may be seamlessly linked}. When the optional sequence (13) does not have a base, the target region (121) and the region (111) that matches the target sequence on the complementary strand are deleted in their entirety. This is how a genome is edited using the single-stranded polynucleotide, applying the PODiR mechanism. A cell whose genome has been edited can be obtained, for example, by cloning and analyzing the sequence of a region that includes the target region on the genome.

In a preferred aspect, as shown in Fig. 9B, the step (A) is performed in the presence of either or both a single-stranded antisense polynucleotide (20) and a single-stranded antisense polynucleotide (30). The antisense polynucleotide (20) has a sequence capable of hybridizing to a region (51) and its upstream region (53), wherein the region (51) is a partial region of RNA (50) transcribed from the genomic region (120), and has a sequence that matches the 5' terminal part of the genome-editing sense strand polynucleotide. The antisense polynucleotide (30) has a sequence capable of hybridizing to a region (52) and its downstream region (54), wherein the region (52) is a partial region of RNA (50) transcribed from the genomic region (120), and has a sequence that matches the 3' terminal part of the genome-editing sense strand polynucleotide.

In a preferred aspect, the lengths of the region (51) and the region (52) can be 1 to 20 bases, for example, can be 2 to 19 bases, can be 3 to 18 bases, can be 4 to 17 bases, can be 5 to 16 bases, can be 6 to 15 bases, can be 7 to 14 bases, can be 8 to 13 bases, can be 9 to 12 bases, can be 10 bases, or 11 bases, respectively. Therefore, the lengths of a region (21) of the single-stranded antisense polynucleotide (20) and a region (31) of the single-stranded antisense polynucleotide (30) that bind to these regions can also be 1 to 20 bases, for example, can be 2 to 19 bases, can be 3 to 18 bases, can be 4 to 17 bases, can be 5 to 16 bases, can be 6 to 15 bases, can be 7 to 14 bases, can be 8 to 13 bases, can be 9 to 12 bases, can be 10 bases, or 11 bases, respectively.

In a preferred aspect, the lengths of the upstream homology arm and the downstream homology arm can be 10 nucleotides or more, 20 nucleotides or more, 30 nucleotides or more, 40 nucleotides or more, 50 nucleotides or more, 60 nucleotides or more, 70 nucleotides or more, 80 nucleotides or more, 90 nucleotides or more, or 100 nucleotides or more, respectively. In an aspect, the lengths of the upstream homology arm and the downstream homology arm can be 200 nucleotides or less, 190 nucleotides or less, 180 nucleotides or less, 170 nucleotides or less, 160 nucleotides or less, 150 nucleotides or less, 140 nucleotides or less, 130 nucleotides or less, 120 nucleotides or less, 110 nucleotides or less, 100 nucleotides or less, 90 nucleotides or less, 80 nucleotides or less, 70 nucleotides or less, 60 nucleotides or less, 50 nucleotides or less, or 40 nucleotides or less, respectively. In an aspect, the lengths of the upstream homology arm and the downstream homology arm can be 10 to 200 nucleotides, 20 to 150 nucleotides, 30 to 100 nucleotides, 35 to 70 nucleotides, or 40 to 50 nucleotides, respectively. The upstream homology arm and the downstream homology arm may be of different or identical lengths. With respect to the upstream homology arm and the downstream homology arm, the shorter arm can be 50% or more, 55% or more, 60% or more, 65% or more, 70% or more, 75% or more, 80% or more, 85% or more, 90% or more, or 95% or more of the length of the longer arm.

In a preferred aspect, the lengths of the antisense polynucleotide (20) and the antisense polynucleotide (30) can be independently more than 10 bases, more than 15 bases, more than 20 bases, more than 25 bases, more than 30 bases, more than 35 bases, or 40 bases or more. In a more preferred aspect, the lengths of the antisense polynucleotide (20) and the antisense polynucleotide (30) can be independently 50 bases or more, 60 bases or more, 70 bases or more, 80 bases or more, 90 bases or more, 100 bases or more, 110 bases or more, 120 bases or more, or 130 nucleotides or more, respectively. The lengths of the antisense polynucleotide (20) and the antisense polynucleotide (30) can independently be, for example, 200 bases or less, 190 bases or less, 180 bases or less, 170 bases or less, 160 bases or less, 150 bases or less, 140 bases or less, or 130 bases or less. The antisense polynucleotide (20) and the antisense polynucleotide (30) may be of identical lengths. The length of the antisense polynucleotide (20) can be within ±50%, ±40%, ±30%, ±25%, ±20%, ±15%, ±10%, or ±5% of the length of the antisense polynucleotide (30). The length of the antisense polynucleotide (30) can be within ±50%, ±40%, ±30%, ±25%, ±20%, ±15%, ±10%, or ±5% of the length of the antisense polynucleotide (20).

Although not bound by any theory, in this way, when RNA (50) is present, the hybridization of RNA (50) to the antisense strand (120) of the genome can reduce the effect that prevents the genome-editing sense polynucleotide (10) from binding to the antisense strand (120). This makes it easier for the genome-editing sense polynucleotide (10) to bind to the antisense strand (120).

In an aspect of the present invention, the step (A) is performed in the presence of at least two types of the single-stranded antisense polynucleotides capable of hybridizing, at least one type of the antisense polynucleotide among them has a sequence capable of hybridizing to a region which is a partial region of RNA transcribed from the genomic region and which has a sequence that matches the 5' terminal part of the genome-editing sense strand polynucleotide, and at least one type of the antisense polynucleotide among them has a sequence capable of hybridizing to a region which is a partial region of RNA transcribed from the genomic region and which has a sequence that matches the 3' terminal part of the genome-editing sense strand polynucleotide. This makes it easier for the genome-editing sense polynucleotide (10) to bind to the antisense strand (120) than when one type of the single-stranded antisense polynucleotide is used.

The genome-editing sense polynucleotide contains, as a constituent unit, for example, a nucleotide selected from the group consisting of DNA, RNA and their derivatives. The derivatives can be a modified nucleotide or a modified nucleic acid. Examples of a modified nucleic acid include a fluorophore-modified nucleic acid, a biotinylated nucleic acid, and a nucleic acid to which a cholesteryl group has been introduced. RNA may be modified at its base with 2'-O-methyl, 2'-fluoro, or 2'-methoxyethyl (MOE) to increase stability. A phosphodiester bond in the nucleic acid backbone of RNA may be replaced with a phosphorothioate bond. Examples of an artificial nucleic acid include a nucleic acid in which the oxygen atom at the 2' position and the carbon atom at the 4' position are cross-linked. Examples of such an artificial nucleic acid include a locked nucleic acid (LNA) that is a cross-linked DNA in which the oxygen atom at the 2' position and the carbon atom at the 4' position are cross-linked through a methylene bridge, ENA in which the oxygen atom at the 2' position and the carbon atom at the 4' position are cross-linked through an ethylene bridge, BNA (Bridged Nucleic Acid) ^{COC} in which the oxygen atom at the 2' position and the carbon atom at the 4' position are cross-linked through -CH₂OCH₂-, a cross-linked nucleic acid (BNA) such as BNA^{NC} in which the oxygen atom at the 2' position and the carbon atom at the 4' position are cross-linked through -NR-CH₂- (R is methyl or a hydrogen atom), cMOE in which the oxygen atom at the 2' position and the carbon atom at the 4' position are cross-linked through - CH₂(OCH₃)-, cEt in which the oxygen atom at the 2' position and the carbon atom at the 4' position are cross-linked through -CH₂(CH₃)-, AmNA in which the carbon atoms at the 2' position and the 4' position are cross-linked through an amide bridge, scpBNA in which the oxygen atom at the 2' position and the carbon atom at the 4' position are cross-linked through a methylene bridge to form a cyclopropane at the 6' position, and a peptide nucleic acid (PNA) whose main chain is an amide-linked polymer of N-(2-aminoethyl)glycine instead of deoxyribose or ribose. The genome-editing sense polynucleotide may also be a DNA-RNA hybrid molecule.

The binding between DNA and RNA is stronger than the binding between DNA and DNA. Considering this, as the antisense polynucleotide (20) and the antisense polynucleotide (30), a polynucleotide with a strong binding affinity for RNA can be used with a view to improving the affinity for RNA, and the polynucleotide can be, for example, DNA, LNA, BNA, and morpholino oligo, or a combination of them. As the genome-editing sense polynucleotide, a polynucleotide with a strong binding affinity for DNA can be used with a view to improving binding to DNA, and it can be a polynucleotide containing, for example, a constituent unit selected from the group consisting of RNA, LNA, BNA, and a modified RNA, or a combination of them.

As a result, the method of the invention allows editing the genome in eukaryotic cells. Specifically, the target region (121) of the genome can be replaced with the sequence of the complementary strand of the optional sequence (13) between the upstream homology arm (11) and the downstream homology arm (12), and the sequence of the region (111) that matches the region on the complementary strand of the target region (121) can be replaced with the optional sequence (13). When the optional sequence (13) is not present, the target region (121) of the genome and the region (111) that matches the region on the complementary strand are deleted.

In an aspect, the target region is located in a region where RNA is transcribed. The region where RNA is transcribed can be a region where transcription activity is activated. In an aspect, the target region can be an exon part. In an aspect, the target region can be an intron part. The size of the target region is not particularly limited, and can be, for example, 10kbp or less, 9kbp or less, 8kbp or less, 7kbp or less, 6kbp or less, 5kbp or less, 4kbp or less, 3kbp or less, 2kbp or less, 1kbp or less, 900bp or less, 800bp or less, 700bp or less, 600bp or less, 500bp or less, 400bp or less, 300bp or less, 200bp or less, 100bp or less, 90bp or less, 80bp or less, 70bp or less, 60bp or less, 50bp or less, 40bp or less, 30bp or less, 20bp or less, 10bp or less, 9bp or less, 8bp or less, 7bp or less, 6bp or less, 5bp or less, 4bp or less, 3bp or less, 2bp or less, 1 bp or less, or 0 bp {0bp can be set, for example, when inserting a sequence into the target region}.

In an aspect, the methods of the present invention do not involve cleavage of the target region by an exogenous DNA endonuclease. In an aspect, the methods of the present invention do not use an exogenous DNA endonuclease in editing and targeting the target region. In an aspect, the methods of the present invention do not use ZFN, TALEN and CRISPR/Cas systems in editing and targeting of the target region. In an aspect, the methods of the present invention may be used in combination with other genome editing tools to edit a region other than the target region.

According to the present invention, there is provided a method for targeting a target region of a genome, including:
the step (A) of causing a single-stranded genome-editing sense polynucleotide to interact with a genome in the cell in a sequence-specific manner,
wherein the genome-editing sense polynucleotide is capable of hybridizing to a sequence adjacent upstream and a sequence adjacent downstream of a target region that matches the antisense strand of a genomic region where RNA is transcribed,
the single-stranded genome-editing sense polynucleotide has an upstream homology arm and a downstream homology arm,
the upstream homology arm has a sequence capable of hybridizing to a sequence adjacent upstream of the target region,
the downstream homology arm has a sequence capable of hybridizing to a sequence adjacent downstream of the target region,
the genome-editing sense polynucleotide can hybridize, through the upstream homology arm and the downstream homology arm, to at least the upstream and downstream of the target region,
the genome-editing sense polynucleotide may have an optional sequence between the upstream homology arm and the downstream homology arm, or may have no sequence between them,
the step (A) is performed in the presence of a single-stranded antisense polynucleotide,
the antisense polynucleotide has a sequence capable of hybridizing to a region which is a partial region of RNA transcribed from the genomic region and which has a sequence that matches the terminal part of the genome-editing sense strand polynucleotide, and
the length of the antisense polynucleotide is 40 nucleotides or more.

A substance (e.g., a label or protein) can be conjugated to the single-stranded genome-editing sense polynucleotide, which allows the substance (e.g., a label or protein) to be recruited (or targeted) to the target region of the genome. The label can be a label such as a fluorescent label, dye, RI label, and tag. In this aspect, the single-stranded genome-editing sense polynucleotide (10) may have a sequence identical to the sequence (111) of the region that matches the target region on the complementary strand of the target region as the optional sequence (13) between the upstream homology arm (11) and the downstream homology arm (12).

According to the present invention, there is provided a kit for genome targeting or a kit for genome editing, containing the single-stranded genome-editing sense polynucleotide (10), and at least one type, preferably both, of the single-stranded antisense polynucleotide (20) and the single-stranded antisense polynucleotide (30). The single-stranded genome-editing sense polynucleotide (10), the single-stranded antisense polynucleotide (20) and the single-stranded antisense polynucleotide (30) are as described above.

According to the present invention, there is provided a kit for genome targeting or a kit for genome editing, containing a polynucleotide encoding the single-stranded genome-editing sense polynucleotide (10), and at least one type, preferably both, of the single-stranded antisense polynucleotide (20) and the single-stranded antisense polynucleotide (30). The polynucleotide encoding the single-stranded genome-editing sense polynucleotide (10) is operably linked to a regulatory sequence. This allows these polynucleotides to be expressible.

According to the present invention, there is provided a kit for genome targeting or a kit for genome editing, containing the single-stranded genome-editing sense polynucleotide (10), and at least one type, preferably both, of a polynucleotide encoding the single-stranded antisense polynucleotide (20) and a polynucleotide encoding the single-stranded antisense polynucleotide (30). Each of the polynucleotide encoding the single-stranded antisense polynucleotide (20) and the polynucleotide encoding the single-stranded antisense polynucleotide (30) is operably linked to a regulatory sequence. This allows these polynucleotides to be expressible.

According to the present invention, there is provided a kit for genome targeting or a kit for genome editing, containing a polynucleotide encoding the single-stranded genome-editing sense polynucleotide (10), and at least one type, preferably both, of a polynucleotide encoding the single-stranded antisense polynucleotide (20) and a polynucleotide encoding the single-stranded antisense polynucleotide (30). Each of the polynucleotide encoding the single-stranded genome-editing sense polynucleotide (10), the polynucleotide encoding the single-stranded antisense polynucleotide (20) and the polynucleotide encoding the single-stranded antisense polynucleotide (30) is operably linked to a regulatory sequence. This allows these polynucleotides to be expressible.

In the above aspects, each of the polynucleotide encoding the single-stranded genome-editing sense polynucleotide (10), the polynucleotide encoding the single-stranded antisense polynucleotide (20) and the polynucleotide encoding the single-stranded antisense polynucleotide (30) is operably linked to a regulatory sequence, and can be contained in one or more expression vectors.

In the above aspects, the kit for genome targeting can preferably be used in the method for targeting a genome of the present invention. In the kit for genome targeting, the single-stranded genome-editing sense polynucleotide (10) is RNA, and can target the target region of the genome in the form of RNA.

In the above aspects, the kit for genome editing can preferably be used in the method for editing a genome of the present invention. The kit for genome editing may further contain a transfection reagent for introducing a polynucleotide into a cell.

In all of the above aspects, the single-stranded genome-editing sense polynucleotide (10), the single-stranded antisense polynucleotide (20), and the single-stranded antisense polynucleotide (30) may be provided in the form of being incorporated into a single strand. Thus, according to the present invention, there is provided a single-stranded chimeric polynucleotide, including the single-stranded antisense polynucleotide (20), the single-stranded genome-editing sense polynucleotide (10), and the single-stranded antisense polynucleotide (30) in this order. According to the present invention, there is also provided an expression vector containing DNA encoding the single-stranded chimeric polynucleotide in an expressible manner.

According to the present invention, there is also provided a cell whose genome has been modified by the method for editing a genome of the present invention, and an organism containing the cell.

### [Examples]

### Example 1: Genome Editing By Genome-Editing Sense Polynucleotide And Promoting Antisense Polynucleotide For Genome Editing

A single-stranded sense strand polynucleotide which directly contributes to genome editing (namely, a genome-editing sense polynucleotide), binds to genomic DNA and modifies the genomic DNA to the sequence of the genome-editing sense polynucleotide. The genome-editing sense polynucleotide hybridizes with the DNA on the antisense side of genomic DNA, which is the first step in genome editing. However, when targeting a transcribed region of genomic DNA, RNA such as newly synthesized mRNA, or fragments thereof (e.g., fragments that are removed by splicing), hybridize on the DNA, and thus may inhibit hybridization of the genome-editing sense polynucleotide to the antisense side of the DNA. In particular, the experiments by the inventors have shown that genome-editing targeting introns is less efficient than genome-editing targeting exons. In introns, RNA fragments from mRNAs that have been processed and excised may bind to the relevant intron parts of genomic DNA and inhibit genome editing.

An antisense polynucleotide for detachment was designed with the aim to detach this newly synthesized RNA hybridized to the antisense strand of the genomic DNA from the genomic DNA. The antisense polynucleotide for detachment was designed to bind to a DNA region that partially overlaps the DNA region where the genome-editing sense polynucleotide hybridizes. The synthesized antisense polynucleotide for detachment was introduced into the system, and genome editing with the genome-editing sense polynucleotide was tested in the presence of the antisense polynucleotide for detachment.

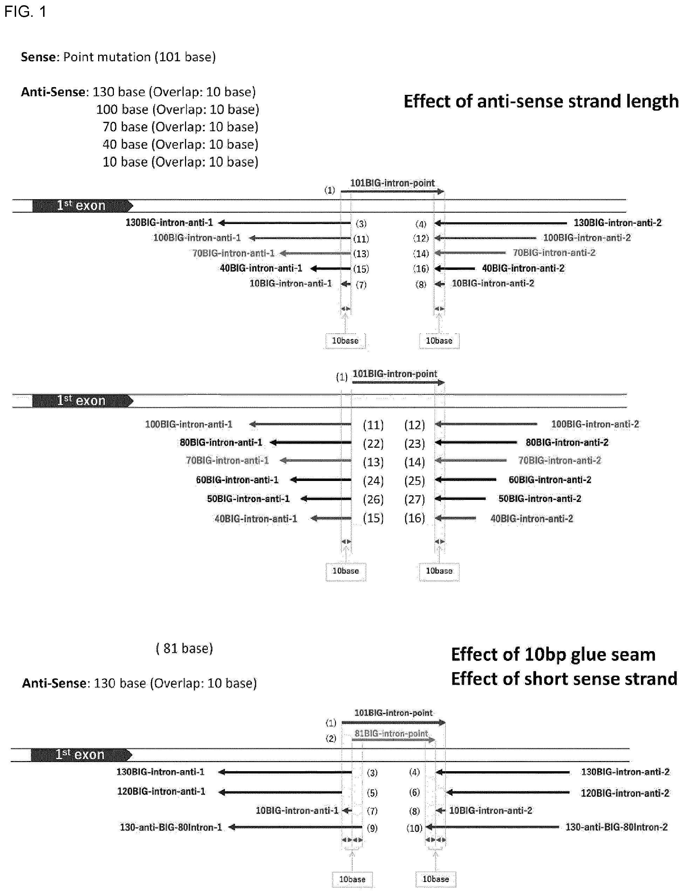

Specifically, a genome-editing sense polynucleotide (101 bases long) represented by (1) was prepared as shown in Fig. 1. In addition, antisense polynucleotides for detachment represented by (3), (4), (7), (8), (11) to (16), and (22) to (27) were prepared as shown in Fig. 1. The antisense polynucleotides for detachment represented by (3), (4), (7), (8), (11) to (16), and (22) to (27) were designed to have a sequence that is capable of hybridizing to the terminal 10 bases of the genome-editing sense polynucleotide represented by (1) (which may be referred to as the "glue seam"). (3) and (4) are 130 bases long, (7) and (8) are 10 bases long, (11) and (12) are 100 bases long, (13) and (14) are 70 bases long, (15) and (16) are 40 bases long, (22) and (23) are 80 bases long, (24) and (25) are 60 bases long, and (26) and (27) are 50 bases long.

A genome-editing sense polynucleotide (81 bases long) represented by (2) was also prepared as shown in Fig. 1. Next, antisense polynucleotides for detachment represented by (5) and (6), which are 120 bases long and designed to hybridize to positions that do not overlap with (1), and antisense polynucleotides for detachment represented by (9) and (10), which are 130 bases long and designed to have a sequence that is capable of hybridizing to the 10 bases at the terminal of (2), were prepared.

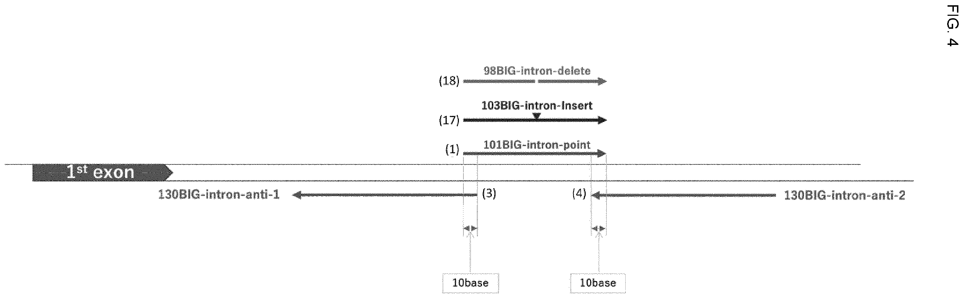

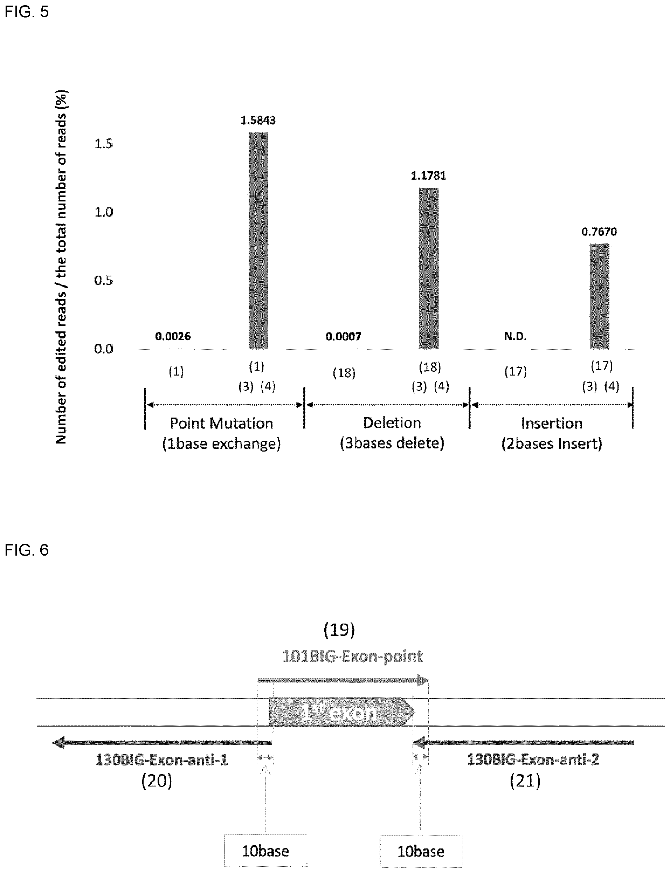

Furthermore, genome-editing sense polynucleotides represented by (17) and (18) were prepared as shown in Fig. 4. In addition, the genome-editing sense polynucleotide represented by (19) and antisense polynucleotides for detachment represented by (20) and (21) were prepared as shown in Fig. 6.

Polynucleotides (1) to (18) and (22) to (27) described above are set within the first intron in the region encoding ARFGEF3 (BIG3) in human genomic DNA. Genome-editing sense polynucleotide (1) and (2) have a nucleotide sequence which is a partial sequence on the sense strand side of the above intron with substitution of one nucleotide, genome-editing sense polynucleotide (17) has a nucleotide sequence which is a partial sequence on the sense strand side of the above intron with insertion of three nucleotides, and genome-editing sense polynucleotide (18) has a nucleotide sequence which is a partial sequence on the sense strand side of the above intron with deletion of two nucleotides. Antisense polynucleotides for detachment (3) to (18) have a partial sequence on the antisense side of the antisense strand of the above intron.

The 10 nucleotides at the 5' terminal side of genome-editing sense polynucleotides (1), (17), and (18) are complementary to the 10 nucleotides at the 5' terminal side of antisense polynucleotides for detachment (3), (11), (13), (15), (22), (24), and (26), and to antisense polynucleotide for detachment (7). The 10 nucleotides at the 3' terminal side of genome-editing sense polynucleotides (1), (17), and (18) are complementary to the 10 nucleotides at the 3' terminal side of antisense polynucleotides for detachment (4), (12), (14), (16), (23), (25), and (27), and to antisense polynucleotide for detachment (8).

The 10 nucleotides at the 5' terminal side of genome-editing sense polynucleotide (2) are complementary to the 10 nucleotides of the 5' terminal side of antisense polynucleotide for detachment (9), and the 10 nucleotides at the 3' terminal side of genome-editing sense polynucleotide (2) are complementary to the 10 nucleotides at the 3' terminal side of antisense polynucleotide for detachment (10).

Genome-editing sense polynucleotide (1) and antisense polynucleotides for detachment (5) and (6) do not have complementary sequences, and genome-editing sense polynucleotide (2) and antisense polynucleotides for detachment (3) and (4) do not have complementary sequences.

Genome-editing sense polynucleotide (19) is set at the position that covers the whole first exon in the region encoding ARFGEF3 (BIG3) in human genomic DNA, and the polynucleotide has a nucleotide sequence which is a sequence on the sense strand side at the position with substitution of one nucleotide. Antisense polynucleotide for detachment (20) is set at the position that covers a part of the untranslated region on the 5' terminal side and a part of the first exon in the region encoding ARFGEF3, and antisense polynucleotide for detachment (21) is set within the first intron in the region encoding ARFGEF3. These antisense polynucleotides have partial sequences on the antisense strand side.

The 10 nucleotides at the 5' terminal side of genome-editing sense polynucleotides (19) are complementary to the 10 nucleotides at the 5' terminal side of antisense polynucleotides for detachment (20), and the 10 nucleotides at the 3' terminal side of genome-editing sense polynucleotides (19) are complementary to the 10 nucleotides at the 3' terminal side of antisense polynucleotides for detachment (21).

The specific sequences and sequence numbers of the above polynucleotides are shown in the table below.

**[Table 2]**

| Name | Nucleotide sequence (5'→3) | SEQ ID NO: |
|---|---|---|
| polynucleotide (1) | tgtcctccctgcctggcggcccccttctcctggtcccggcgctgggg acgCtgcttccccatcgccgagttaggcaggacccggagccggt gcgaatcctg | 1 |
| polynucleotide (2) | gcctggcggcccccttctcctggtcccggcgctggggacgCtgctt ccccatcgccgagttaggcaggacccggagccggt | 2 |
| polynucleotide (3) | agggaggacagcggttcccgaggaaggtttcggagagtgaaag agaccggaggggaggcggctgatgtccacgcccgagccaaga ctctcaaatcccacatcaatttcccgaggcgactctcgcaatcgc | 3 |
| polynucleotide (4) | ctctatcaggaaaatcaaattaaacaagcctttccttcctggttccac tcgccaaggattcgacatcggggttgttggtttaacttcccttttcccc agaggcgcttcgctgtcaggccccaggattcgc | 4 |
| polynucleotide (5) | gcggttcccgaggaaggtttcggagagtgaaagagaccggagg ggaggcggctgatgtccacgcccgagccaagactctcaaatccc acatcaatttcccgaggcgactctcgcaatcgc | 5 |
| polynucleotide (6) | ctctatcaggaaaatcaaattaaacaagcctttccttcctggttccac tcgccaaggattcgacatcggggttgttggtttaacttcccttttcccc agaggcgcttcgctgtcaggccc | 6 |
| polynucleotide (7) | agggaggaca | 7 |
| polynucleotide (8) | caggattcgc | 8 |
| polynucleotide (9) | gccgccaggcagggaggacagcggttcccgaggaaggtttcgg agagtgaaagagaccggaggggaggcggctgatgtccacgcc cgagccaagactctcaaatcccacatcaatttcccgaggcgactc | 9 |
| polynucleotide (10) | aaaatcaaattaaacaagcctttccttcctggttccactcgccaagg attcgacatcggggttgttggtttaacttcccttttccccagaggcgctt cgctgtcaggccccaggattcgcaccggctccg | 10 |
| polynucleotide (11) | agggaggacagcggttcccgaggaaggtttcggagagtgaaag agaccggaggggaggcggctgatgtccacgcccgagccaaga ctctcaaatcccaca | 11 |
| polynucleotide (12) | tttccttcctggttccactcgccaaggattcgacatcggggttgttggtt taacttcccttttccccagaggcgcttcgctgtcaggccccaggattc gc | 12 |
| polynucleotide (13) | agggaggacagcggttcccgaggaaggtttcggagagtgaaag agaccggaggggaggcggctgatgtcc | 13 |
| polynucleotide (14) | cgacatcggggttgttggtttaacttcccttttccccagaggcgcttcg ctgtcaggccccaggattcgc | 14 |
| polynucleotide (15) | agggaggacagcggttcccgaggaaggtttcggagagtga | 15 |
| polynucleotide (16) | tttccccagaggcgcttcgctgtcaggccccaggattcgc | 16 |
| polynucleotide (17) | tgtcctccctgcctggcggcccccttctcctggtcccggcgctgggg acgTGCtgcttccccatcgccgagttaggcaggacccggagcc ggtgcgaatcctg | 17 |
| polynucleotide (18) | tgtcctccctgcctggcggcccccttctcctggtcccggcgctgggg acgcttccccatcgccgagttaggcaggacccggagccggtgcg aatcctg | 18 |
| polynucleotide (19) | aggtcaagatggaagaaatcctgaggaagctgcagaaggagg cgtccgggCgcaagtacaaagccatcaaggagagctgcacctg ggccctgggtaagcgt | 19 |
| polynucleotide (20) | atcttgaccttccaggcgccgggccgccgcccacagggagaga agcgcgggcgagccgggccggggaagccgccgctgcctggcc ctgggcgcctaggcggccgccgcgcgcgcccccatgccgcccc gg | 20 |
| polynucleotide (21) | ggcgactctcgcaatcgccagaacggcacccatgaccgacgctc cgcgaaagccccacgcgcggaggcgcgggttcagccccggcc gcgcggccctcccgccgcggcgagcaggtgccggacgcttacc c | 21 |
| polynucleotide (22) | agggaggacagcggttcccgaggaaggtttcggagagtgaaag agaccggaggggaggcggctgatgtccacgcccgagc | 22 |
| polynucleotide (23) | gccaaggattcgacatcggggttgttggtttaacttcccttttccccag aggcgcttcgctgtcaggccccaggattcgc | 23 |
| polynucleotide (24) | agggaggacagcggttcccgaggaaggtttcggagagtgaaag agaccggaggggaggcg | 24 |
| polynucleotide (25) | gttgttggtttaacttcccttttccccagaggcgcttcgctgtcaggcc ccaggattcgc | 25 |
| polynucleotide (26) | agggaggacagcggttcccgaggaaggtttcggagagtgaaag agaccgg | 26 |
| polynucleotide (27) | taacttcccttttccccagaggcgcttcgctgtcaggccccaggattc gc | 27 |

In the above table, capital letters are the parts of the genome sequence that differ from the sequence of the genome to be edited.

Thus, genome-editing was induced in cells using genome-editing sense polynucleotides in the presence of antisense polynucleotides for detachment.

Specifically, the study was performed as follows.
[1] First, HEK293T cells were cultured in Dulbecco's Modified Eagle's Medium-high glucose; DMEM (Sigma, D5796) containing 10% (v/v) Fetal Bovine Serum (Sigma, F7524) and 1% (v/v) Antibiotic-Antimycotic (Gibco, 15240-062) until confluent cells in 10 cm dishes.
[2] Next, the supernatant was removed using an aspirator, and 1 mL PBS (-) (FUJIFILM Wako Pure Chemicals, 166-23555) and 1 mL of 0.05% Trypsin-EDTA (Gibco, 25300-062) were added, and the cells were treated at 37°C for 3 min.
[3] Next, 3.6 mL DMEM (Sigma, D5796), 0.36 mL Fetal Bovine Serum (Biowest, S1760) and 0.04 mL Antibiotic-Antimycotic (Gibco, 15240-062) were added, and the cells were detached from the plate by pipetting back. After the cells were collected in a 15-mL centrifuge tube, they were centrifuged at 1000 rpm for 5 min, the cells were collected at the bottom of the centrifuge tube, and the supernatant was removed using an aspirator.
[4] Then, 4.5 mL DMEM (Sigma, D5796), 0.45 mL Fetal Bovine Serum (Sigma, F7524), and 0.05 mL Antibiotic-Antimycotic (Gibco, 15240-062) were added, and the cells were resuspended.
[5] The number of cells in the suspension was measured, and the cells were dispensed into 6-well multi-well plates (Corning, 3516) to be 2.5 × 10⁵ cells/well.
[6] The cells were incubated in a CO₂ incubator at 37°C for 2 days until 60-80% confluent cells.
[7] Then, 150 µl of DNA mixture solution (Opti-MEM (Gibco, 11058-021) + Fugene HD solution) containing 5 µg of each polynucleotide was added, and incubated at 37°C for 1 day in a CO₂ incubator.
[8] The supernatant of HEK293T cells was removed using an aspirator, and then 0.3 mL PBS (-) (Fujifilm Wako Pure Chemicals, 166-23555) and 0.3 mL of 0.05% Trypsin-EDTA (Gibco, 25300-062) were added, and the cells were treated at 37°C for 3 min.
[9] Next, 2.7 mL DMEM (Sigma, D5796), 0.27 mL Fetal Bovine Serum (Biowest, S1760) and 0.03 mL Antibiotic-Antimycotic (Gibco, 15240 -062) were added, and the cells were detached from the plate by pipetting back, collected in a 15-mL centrifuge tube. The cells were collected at 1000 rpm, 5 min at the bottom of the centrifuge tube, and the supernatant was removed using an aspirator.
[10] Then, 9 mL DMEM (Sigma, D5796), 0.9 mL Fetal Bovine Serum (Sigma, F7524), and 0.1 mL Antibiotic-Antimycotic (Gibco, 15240-062) were added, and the cells were resuspended and seeded into 10-cm dishes.
[11] The cells were incubated in a CO₂ incubator at 37°C for 4 days to allow sufficient growth.
[12] The supernatant of HEK293T cells was removed using an aspirator, and 1 mL PBS (-) (FUJIFILM Wako Pure Chemicals, 166-23555) and 1 mL of 0.05% Trypsin-EDTA (Gibco, 25300-062) were added, and the cells were treated at 37°C for 3 min.
[13] Next, 3.6 mL DMEM (Sigma, D5796), 0.36 mL Fetal Bovine Serum (Biowest, S1760) and 0.04 mL Antibiotic-Antimycotic (Gibco, 15240 -062) were added, and the cells were detached from the plate by pipetting back, collected in a 15-mL centrifuge tube. The cells were collected at 1000 rpm, 5 min at the bottom of the centrifuge tube, and the supernatant was removed using an aspirator.
[14] Then, 4.5 mL DMEM (Sigma, D5796), 0.45 mL Fetal Bovine Serum (Sigma, F7524), and 0.05 mL Antibiotic-Antimycotic (Gibco, 15240-062) were added, and the cells were resuspended.
[15] The number of cells in the suspension was measured, and the suspension of 2.0 × 10⁶ cells was transferred to a 1.5-mL tube, and the cells were collected at 1000 rpm for 5 min at the bottom of the tube, and the supernatant was removed using an aspirator.
[16] Genome DNA was extracted from the cell pellet (2.0 × 10⁶ cells) remaining in the 1.5-mL tube using a genome extraction kit (NucleoSpin Tissue Kit) manufactured by Macherey-Nagel, Inc. The genome-editing efficiency was calculated as the number of edited reads to the total number of reads by determining the percentage of genomes edited as designed using a next-generation sequencer.

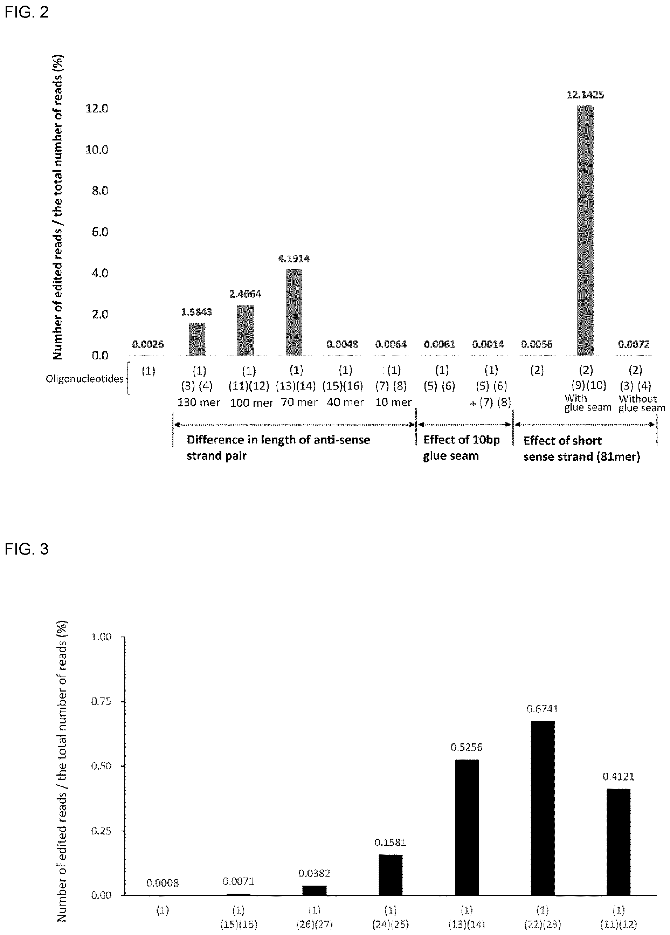

The results are as shown in Fig. 2. As shown in Fig. 2, the genome-editing efficiency using the genome-editing sense polynucleotide represented by (1) in the absence of antisense polynucleotides for detachment was 0.0026%. On the other hand, the genome-editing efficiency was markedly improved in the presence of the combinations of antisense polynucleotides for detachment represented by (3) and (4), (11) and (12), and (13) and (14).

As further shown in Fig. 2, the genome-editing efficiency with the genome-editing sense polynucleotide represented by (2) was 0.0056%, while the genome-editing efficiency in the presence of antisense polynucleotides for detachment (9) and (10) was 12.1425%.

Genome editing using genome-editing sense polynucleotide (1) in the presence of antisense polynucleotides for detachment (11) to (16) and (22) to (27) was also performed using the same procedure except that the amount of each polynucleotide in the DNA mixture solution used in [7] in the above test procedure was changed from 5 µg to 50 pmol. As shown in Fig. 3, the genome-editing efficiency of genome-editing sense polynucleotide (1) was improved in the presence of each combination of antisense polynucleotides for detachment. In particular, the improvement in genome-editing efficiency was particularly pronounced in the presence of the combinations of (11) and (12), (13) and (14), and (22) and (23).

In the above experiment, genome-editing efficiency was confirmed using eukaryotic cells. As a result, the efficiency of genome-editing by the genome-editing sense polynucleotides was improved in the presence of the antisense polynucleotides for detachment. This suggests that the antisense polynucleotides for detachment eliminated the factor that prevents genome editing by the genome-editing sense polynucleotides. The antisense polynucleotides for detachment were originally designed to prevent RNA transcribed from genomic DNA from binding to DNA. It was suggested that the transcribed RNA was bound to the genomic DNA by DNA-RNA interactions, which may have inhibited the genome-editing sense polynucleotides from hybridizing to the relevant region. Since DNA-RNA interactions are more robust than DNA-DNA interactions, the inhibitory effect is considered to be particularly strong when DNA is used as the genome-editing sense polynucleotides.

Furthermore, as shown in Fig. 4, the same experiment as above was performed using genome-editing sense polynucleotide (18) with base deletion and genome-editing sense polynucleotide (17) with base insertion, instead of genome-editing sense polynucleotide (1) with point mutation. The results are as shown in Fig. 5. As shown in Fig. 5, both genome-editing sense polynucleotide (18) with base deletion and genome-editing sense polynucleotide (17) with base insertion showed that the editing efficiency was greatly improved in the presence of antisense polynucleotides.

As shown in Fig. 6, the same experiment as above was performed using genome-editing sense polynucleotide (19) in the presence of antisense polynucleotides (20) and (21) to target exon. The results are as shown in Fig. 7. As shown in Fig. 7, in the presence of both-sided antisense polynucleotides (antisense polynucleotides (20) and (21)), the editing efficiency by the genome-editing sense polynucleotide was greatly improved compared to that in the absence of the antisense polynucleotides even when targeting exon. In addition, as shown in Fig. 7, the editing efficiency in the presence of only one-sided antisense polynucleotide (21) also showed approximately 7- to 8-fold improvement compared to that in the absence of the antisense polynucleotide. This reveals that an antisense polynucleotide designed in a position partially overlapping with the sense strand significantly improves editing efficiency, even if only on one side.

These results demonstrated that an antisense polynucleotide designed to bind to a DNA region that partially overlaps a DNA region where the genome-editing sense polynucleotide hybridizes can be utilized as a promoting antisense polynucleotide for genome editing.

## Claims

1. A single-stranded promoting polynucleotide for genome editing for use in combination with a single-stranded genome-editing polynucleotide capable of modifying a target site on a double-stranded genomic DNA,
wherein the target site consists of a primary editing site set on one genomic DNA strand and a secondary editing site on the other genomic DNA strand at a position corresponding to the primary editing site,
the genome-editing polynucleotide consists of, in order from its 5' terminal side,
a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b),
the lengths of portions a) and b) are each independently 10 to 100 nucleotides,
the promoting polynucleotide for genome editing is capable of hybridizing to a nucleic acid sequence that is complementary to a predetermined region on the one genomic DNA strand,
the predetermined region overlaps at its 5' terminal part with region A or overlaps at its 3' terminal part with region B on the one genomic DNA strand, in which a region on the one genomic DNA strand at a position corresponding to portion a) of the genome-editing polynucleotide is defined as region A, and a region on the one genomic DNA strand at a position corresponding to portion b) of the genome-editing polynucleotide is defined as region B,
the length of overlap with region A at the 5' terminal part of the predetermined region is the same as or shorter than region A, and the length of overlap with region B at the 3' terminal part of the predetermined region is the same as or shorter than region B,
the length of the promoting polynucleotide for genome editing is 50 nucleotides or more, and
when the genome-editing polynucleotide consists of portion a), portion x), and portion b), and the primary editing site is set between two contiguous nucleotides on the one genomic DNA strand, the genome-editing polynucleotide can insert a portion consisting of a nucleotide sequence complementary to portion x) into the primary editing site and can insert portion x) into the secondary editing site,
when the genome-editing polynucleotide consists of portion a), portion x), and portion b), and the primary editing site is set at one nucleotide or multiple contiguous nucleotides on the one genomic DNA strand, the genome-editing polynucleotide can replace the primary editing site with a portion consisting of a nucleotide sequence complementary to portion x) and can replace the secondary editing site with portion x), and
when the genome-editing polynucleotide consists of portion a) and portion b), and the primary editing site is set at one nucleotide or multiple contiguous nucleotides on the one genomic DNA strand, the genome-editing polynucleotide can delete the target site.

2. The promoting polynucleotide for genome editing according to claim 1, wherein the length of the overlap is 5 nucleotides or more.

3. A kit for genome editing, comprising:
the promoting polynucleotide for genome editing according to claim 1 or 2,
an expression vector thereof, or
a conjugate of the promoting polynucleotide for genome editing or the expression vector thereof with one or more other substances, and
a genome-editing polynucleotide,
an expression vector thereof, or
a conjugate of the genome-editing polynucleotide or the expression vector thereof with one or more other substances,
wherein the genome-editing polynucleotide consists of, in order from its 5' terminal side,
a) a portion capable of hybridizing to the region adjacent to the 3' terminal side of the primary editing site,
x) an optional portion consisting of a nucleotide sequence that is not identical to the nucleotide sequence of the secondary editing site, and
b) a portion capable of hybridizing to the region adjacent to the 5' terminal side of the primary editing site, or
the genome-editing polynucleotide consists of, in order from its 5' terminal side, portion a) and portion b).

4. The kit for genome editing according to claim 3, comprising:
the promoting polynucleotide for genome editing in which the predetermined region is set to overlap at its 5' terminal part with region A,
an expression vector thereof, or
a conjugate of the promoting polynucleotide for genome editing or the expression vector thereof with one or more other substances, and
the promoting polynucleotide for genome editing in which the predetermined region is set to overlap at its 3' terminal part with region B,
an expression vector thereof, or
a conjugate of the promoting polynucleotide for genome editing or the expression vector thereof with one or more other substances.

5. The kit for genome editing according to claim 3 or 4, wherein the length of the overlap with respect to the promoting polynucleotide for genome editing is 5 nucleotides or more.

6. A method for modifying a target site on a double-stranded genomic DNA of a cell excluding human gametes and fertilized eggs, or a non-human organism, comprising the step of treating the cell excluding human gametes and fertilized eggs or the non-human organism using the kit for genome editing according to any one of claims 3 to 5, provided that when the cell is a human cell excluding human gametes and fertilized eggs, the step is performed in vitro.

7. A method for producing a cell excluding human gametes and fertilized eggs, or a non-human organism, in which a target site on a double-stranded genomic DNA has been modified, comprising the step of treating the cell excluding human gametes and fertilized eggs or the non-human organism using the kit for genome editing according to any one of claims 3 to 5, provided that when the cell is a human cell excluding human gametes and fertilized eggs, the step is performed in vitro.
